(19) Europäisches Patentamt
European Patent Office
Office européen des brevets

(11) **EP 1 295 937 A2**

(12) **EUROPÄISCHE PATENTANMELDUNG**

(43) Veröffentlichungstag:
**26.03.2003 Patentblatt 2003/13**

(51) Int Cl.[7]: **C12N 9/02**, C12N 15/53, C12N 15/63, C12P 41/00, C12N 1/19, C12Q 1/26

(21) Anmeldenummer: **02019948.5**

(22) Anmeldetag: **05.09.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **21.09.2001 DE 10146589**

(71) Anmelder: **Degussa AG**
**40474 Düsseldorf (DE)**

(72) Erfinder:
- **Slusarczyk, Heike, Dr.**
  **52531 Übach-Palenberg (DE)**
- **Felber, Stefan, Dr.**
  **52428 Jülich (DE)**
- **Kula, Maria-Regina, Prof.**
  **81477 München (DE)**
- **Pohl, Martina, Dr.**
  **52066 Aachen (DE)**

(54) **Mutanten der Formiathehydrogenase aus Candida boidinii**

(57) Die vorliegende Erfindung befaßt sich mit rekombinant hergestellten Enzymen, insbesondere mit rec-FDHs. Durch gerichtete Evolution ist es gelungen, katalytisch aktivere und stabilere Muteine zu generieren, welche vorzugsweise in einem technischen Prozeß zur Herstellung von z.B. Aminosäuren eingesetzt werden können.

Weiterhin beschäftigt sich die Erfindung mit den diese Enzyme codierenden Nukleinsäuren, diese aufweisenden . Vehikeln und vorteilhaften Primern zur Herstellung der Nukleinsäuren mittels PCR.

Zusätzlich wird ein Verfahren zur Herstellung weiterhin rec-FDHs genannt, sowie ein Verfahren zum Screenen von stabileren und/oder aktiveren Dehydrogenasen beansprucht.

Fig. 3:

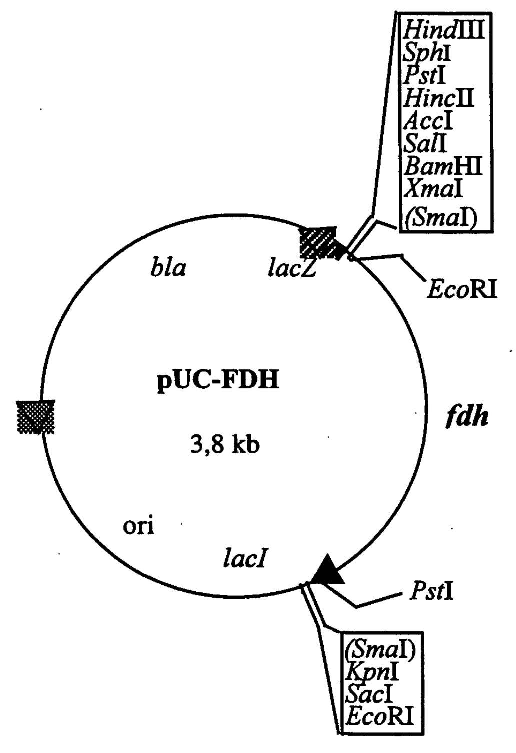


EP 1 295 937 A2

Printed by Jouve, 75001 PARIS (FR)

SEQUENZPROTOKOLL

<110> Degussa AG

<120> Neue Mutanten der Formiatdehydrogenase aus Candida boidinii

<130> 010155 AM

<140>
<141>

<160> 43

<170> PatentIn Ver. 2.1

<210> 1
<211> 1095
<212> DNA
<213> Künstliche Sequenz

<220>
<223> Beschreibung der künstlichen Sequenz:C23S

<220>
<221> CDS
<222> (1)..(1095)

<400> 1

```
atg aag att gtc tta gtt ctt tat gat gct ggt aag cac gct gct gat    48
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat    96
Glu Glu Lys Leu Tyr Gly Ser Thr Glu Asn Lys Leu Gly Ile Ala Asn
             20                  25                  30

tgg tta aaa gat caa ggt cat gaa cta att act act tct gat aaa gaa    144
Trp Leu Lys Asp Gln Gly His Glu Leu Ile Thr Thr Ser Asp Lys Glu
         35                  40                  45

ggt gaa aca agt gaa ttg gat aaa cat atc cca gat gct gat att atc    192
Gly Glu Thr Ser Glu Leu Asp Lys His Ile Pro Asp Ala Asp Ile Ile
     50                  55                  60

atc acc act cct ttc cat cct gct tat atc act aag gaa aga ctt gac    240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat    288
Lys Ala Lys Asn Leu Lys Leu Val Val Val Ala Gly Val Gly Ser Asp
                 85                  90                  95

cac att gat tta gat tat att aat caa aca ggt aag aaa atc tca gtc    336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc    384
Leu Glu Val Thr Gly Ser Asn Val Val Ser Val Ala Glu His Val Val
```

```
        115                 120                 125

atg acc atg ctt gtc ttg gtt aga aat ttc gtt cca gca cat gaa caa    432
Met Thr Met Leu Val Leu Val Arg Asn Phe Val Pro Ala His Glu Gln
    130                 135                 140

att att aac cac gat tgg gag gtt gct gct atc gct aag gat gct tac    480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt    528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta    576
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt    624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc    672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat    720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc    768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa    816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca    864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct    912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa    960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                1095
```

```
Tyr Val Thr Lys Ala Tyr Gly Lys His Asp Lys Lys
        355                 360                 365


<210> 2
<211> 364
<212> PRT
<213> Künstliche Sequenz
<223> Beschreibung der künstlichen Sequenz:C23S

<400> 2
Met Lys Ile Val Leu Val Leu Tyr Asp Ala Gly Lys His Ala Ala Asp
  1               5                  10                  15
Glu Glu Lys Leu Tyr Gly Ser Thr Glu Asn Lys Leu Gly Ile Ala Asn
             20                  25                  30
Trp Leu Lys Asp Gln Gly His Glu Leu Ile Thr Thr Ser Asp Lys Glu
        35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
 65                  70                  75                  80
Lys Ala Lys Asn Leu Lys Leu Val Val Val Ala Gly Val Gly Ser Asp
                 85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Glu Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

<210> 3
<211> 1095
<212> DNA
<213> Künstliche Sequenz

<220>
<223> Beschreibung der künstlichen Sequenz:C23S und E18D

<220>
<221> CDS
<222> (1)..(1095)

<400> 3
```
atg aag att gtc tta gtt ctt tat gat gct ggt aag cac gct gct gat      48
Met Lys Ile Val Leu Val Leu Tyr Asp Ala Gly Lys His Ala Ala Asp
  1               5                  10                  15

gaa gac aaa tta tat ggt tct act gaa aat aaa tta ggt att gct aat      96
Glu Asp Lys Leu Tyr Gly Ser Thr Glu Asn Lys Leu Gly Ile Ala Asn
             20                  25                  30

tgg tta aaa gat caa ggt cat gaa cta att act act tct gat aaa gaa     144
Trp Leu Lys Asp Gln Gly His Glu Leu Ile Thr Thr Ser Asp Lys Glu
         35                  40                  45

ggt gaa aca agt gaa ttg gat aaa cat atc cca gat gct gat att atc     192
Gly Glu Thr Ser Glu Leu Asp Lys His Ile Pro Asp Ala Asp Ile Ile
     50                  55                  60

atc acc act cct ttc cat cct gct tat atc act aag gaa aga ctt gac     240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat     288
Lys Ala Lys Asn Leu Lys Leu Val Val Val Ala Gly Val Gly Ser Asp
                 85                  90                  95

cac att gat tta gat tat att aat caa aca ggt aag aaa atc tca gtc     336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc     384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa     432
Met Thr Met Leu Val Leu Val Arg Asn Phe Val Pro Ala His Glu Gln
    130                 135                 140

att att aac cac gat tgg gag gtt gct gct atc gct aag gat gct tac     480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt     528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta     576
```

```
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt    624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc    672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat    720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc    768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa    816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca    864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct    912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa    960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                1095
Tyr Val Thr Lys Ala Tyr Gly Lys His Asp Lys Lys
        355                 360                 365


<210> 4
<211> 364
<212> PRT
<213> Künstliche Sequenz
<223> Beschreibung der künstlichen Sequenz:C23S und E18D

<400> 4
Met Lys Ile Val Leu Val Leu Tyr Asp Ala Gly Lys His Ala Ala Asp
 1               5                  10                  15
Glu Asp Lys Leu Tyr Gly Ser Thr Glu Asn Lys Leu Gly Ile Ala Asn
            20                  25                  30
```

```
Trp Leu Lys Asp Gln Gly His Glu Leu Ile Thr Thr Ser Asp Lys Glu
            35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
65                  70                  75                      80
Lys Ala Lys Asn Leu Lys Leu Val Val Val Ala Gly Val Gly Ser Asp
                85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Glu Val Ala Ala Ile Ala Lys Asp Ala Tyr
145                 150                 155                     160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                     240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                     320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

<210> 5
<211> 1095
<212> DNA
<213> Künstliche Sequenz

<220>
<223> Beschreibung der künstlichen Sequenz:C23S und K35R

<220>
<221> CDS
<222> (1)..(1095)

<400> 5
atg aag att gtc tta gtt ctt tat gat gct ggt aag cac gct gct gat    48

```
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat    96
Glu Glu Lys Leu Tyr Gly Ser Thr Glu Asn Lys Leu Gly Ile Ala Asn
                 20                  25                  30

tgg tta aga gat caa ggt cat gaa cta att act act tct gat aaa gaa   144
Trp Leu Arg Asp Gln Gly His Glu Leu Ile Thr Thr Ser Asp Lys Glu
             35                  40                  45

ggt gaa aca agt gaa ttg gat aaa cat atc cca gat gct gat att atc   192
Gly Glu Thr Ser Glu Leu Asp Lys His Ile Pro Asp Ala Asp Ile Ile
         50                  55                  60

atc acc act cct ttc cat cct gct tat atc act aag gaa aga ctt gac   240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat   288
Lys Ala Lys Asn Leu Lys Leu Val Val Val Ala Gly Val Gly Ser Asp
                 85                  90                  95

cac att gat tta gat tat att aat caa aca ggt aag aaa atc tca gtc   336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc   384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa   432
Met Thr Met Leu Val Leu Val Arg Asn Phe Val Pro Ala His Glu Gln
    130                 135                 140

att att aac cac gat tgg gag gtt gct gct atc gct aag gat gct tac   480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt   528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta   576
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt   624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc   672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat   720
Val Thr Val Asn Ala Pro Leu His Ala Gly Thr Lys Gly Leu Ile Asn
225                 230                 235                 240
```

```
aag gaa tta tta tct aaa ttt aaa aaa ggt gct tgg tta gtc aat acc     768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa     816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca     864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct     912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa     960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc     1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa     1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                 1095
Tyr Val Thr Lys Ala Tyr Gly Lys His Asp Lys Lys
        355                 360                 365


<210> 6
<211> 364
<212> PRT
<213> Künstliche Sequenz
<223> Beschreibung der künstlichen Sequenz:C23S und K35R

<400> 6
Met Lys Ile Val Leu Val Leu Tyr Asp Ala Gly Lys His Ala Ala Asp
  1               5                  10                  15
Glu Glu Lys Leu Tyr Gly Ser Thr Glu Asn Lys Leu Gly Ile Ala Asn
            20                  25                  30
Trp Leu Arg Asp Gln Gly His Glu Leu Ile Thr Thr Ser Asp Lys Glu
        35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
 65                  70                  75                  80
Lys Ala Lys Asn Leu Lys Leu Val Val Val Ala Gly Val Gly Ser Asp
                85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Glu Val Ala Ala Ile Ala Lys Asp Ala Tyr
```

```
145                 150                 155                 160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

```
<210> 7
<211> 1095
<212> DNA
<213> Künstliche Sequenz

<220>
<223> Beschreibung der künstlichen Sequenz:C23S und
      D149E

<220>
<221> CDS
<222> (1)..(1095)

<400> 7
atg aag att gtc tta gtt ctt tat gat gct ggt aag cac gct gct gat      48
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat      96
Glu Glu Lys Leu Tyr Gly Ser Thr Glu Asn Lys Leu Gly Ile Ala Asn
             20                  25                  30

tgg tta aaa gat caa ggt cat gaa cta att act act tct gat aaa gaa     144
Trp Leu Lys Asp Gln Gly His Glu Leu Ile Thr Thr Ser Asp Lys Glu
         35                  40                  45

ggt gaa aca agt gaa ttg gat aaa cat atc cca gat gct gat att atc     192
Gly Glu Thr Ser Glu Leu Asp Lys His Ile Pro Asp Ala Asp Ile Ile
     50                  55                  60
```

```
atc acc act cct ttc cat cct gct tat atc act aag gaa aga ctt gac    240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat    288
Lys Ala Lys Asn Leu Lys Leu Val Val Val Ala Gly Val Gly Ser Asp
                 85                  90                  95

cac att gat tta gat tat att aat caa aca ggt aag aaa atc tca gtc    336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc    384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa    432
Met Thr Met Leu Val Leu Val Arg Asn Phe Val Pro Ala His Glu Gln
    130                 135                 140

att att aac cac gag tgg gag gtt gct gct atc gct aag gat gct tac    480
Ile Ile Asn His Glu Trp Glu Val Ala Ala Ile Ala Lys Asp Ala Tyr
145                 150                 155                 160

gat atc gaa ggt aaa act atc gct acc att ggt gct ggt aga att ggt    528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta    576
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt    624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc    672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat    720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc    768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa    816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca    864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct    912
Ala Pro Lys Asp His Pro Trp Arg Asp Met Arg Asn Lys Tyr Gly Ala
```

```
290                 295                 300

ggt aat gcc atg act cct cac tac tct ggt act act tta gac gct caa     960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                1095
Tyr Val Thr Lys Ala Tyr Gly Lys His Asp Lys Lys
        355                 360                 365


<210> 8
<211> 364
<212> PRT
<213> Künstliche Sequenz
<223> Beschreibung der künstlichen Sequenz:C23S und
      D149E

<400> 8
Met Lys Ile Val Leu Val Leu Tyr Asp Ala Gly Lys His Ala Ala Asp
  1               5                  10                  15
Glu Glu Lys Leu Tyr Gly Ser Thr Glu Asn Lys Leu Gly Ile Ala Asn
             20                  25                  30
Trp Leu Lys Asp Gln Gly His Glu Leu Ile Thr Thr Ser Asp Lys Glu
         35                  40                  45
Gly Glu Thr Ser Glu Leu Asp Lys His Ile Pro Asp Ala Asp Ile Ile
     50                  55                  60
Ile Thr Thr Pro Phe His Pro Ala Tyr Ile Thr Lys Glu Arg Leu Asp
 65                  70                  75                  80
Lys Ala Lys Asn Leu Lys Leu Val Val Val Ala Gly Val Gly Ser Asp
                 85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Glu Trp Glu Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
```

```
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

<210> 9
<211> 1095
<212> DNA
<213> Künstliche Sequenz

<220>
<223> Beschreibung der künstlichen Sequenz:C23S und E151D

<220>
<221> CDS
<222> (1)..(1095)

<400> 9

```
atg aag att gtc tta gtt ctt tat gat gct ggt aag cac gct gct gat    48
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat    96
Glu Glu Lys Leu Tyr Gly Ser Thr Glu Asn Lys Leu Gly Ile Ala Asn
            20                  25                  30

tgg tta aaa gat caa ggt cat gaa cta att act act tct gat aaa gaa   144
Trp Leu Lys Asp Gln Gly His Glu Leu Ile Thr Thr Ser Asp Lys Glu
        35                  40                  45

ggt gaa aca agt gaa ttg gat aaa cat atc cca gat gct gat att atc   192
Gly Glu Thr Ser Glu Leu Asp Lys His Ile Pro Asp Ala Asp Ile Ile
    50                  55                  60

atc acc act cct ttc cat cct gct tat atc act aag gaa aga ctt gac   240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat   288
Lys Ala Lys Asn Leu Lys Leu Val Val Val Ala Gly Val Gly Ser Asp
                 85                  90                  95

cac att gat tta gat tat att aat caa aca ggt aag aaa atc tca gtc   336
His Ile Asp Leu Asp Tyr Ile Asn Gln Thr Gly Lys Lys Ile Ser Val
            100                 105                 110
```

```
ctg gaa gtt aca ggt tct aat gtt gtc tct gtt gct gaa cac gtt gtc    384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa    432
Met Thr Met Leu Val Leu Val Arg Asn Phe Val Pro Ala His Glu Gln
    130                 135                 140

att att aac cac gat tgg gat gtt gct gct atc gct aag gat gct tac    480
Ile Ile Asn His Asp Trp Asp Val Ala Ala Ile Ala Lys Asp Ala Tyr
145                 150                 155                 160

gat atc gaa ggt aaa act atc gct acc att ggt gct ggt aga att ggt    528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta    576
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt    624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc    672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat    720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc    768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa    816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca    864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct    912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa    960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350
```

```
tac gtt act aaa gct tac ggt aaa cac gat aag aaa taa                  1095
Tyr Val Thr Lys Ala Tyr Gly Lys His Asp Lys Lys
        355                 360                 365

<210> 10
<211> 364
<212> PRT
<213> Künstliche Sequenz
<223> Beschreibung der künstlichen Sequenz:C23S und
      E151D

<400> 10
Met Lys Ile Val Leu Val Leu Tyr Asp Ala Gly Lys His Ala Ala Asp
 1               5                  10                  15
Glu Glu Lys Leu Tyr Gly Ser Thr Glu Asn Lys Leu Gly Ile Ala Asn
            20                  25                  30
Trp Leu Lys Asp Gln Gly His Glu Leu Ile Thr Thr Ser Asp Lys Glu
        35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
65                  70                  75                  80
Lys Ala Lys Asn Leu Lys Leu Val Val Val Ala Gly Val Gly Ser Asp
                85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Asp Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
```

355 360

<210> 11
<211> 1095
<212> DNA
<213> Künstliche Sequenz

<220>
<223> Beschreibung der künstlichen Sequenz:C23S und
R178S

<220>
<221> CDS
<222> (1)..(1095)

<400> 11
```
atg aag att gtc tta gtt ctt tat gat gct ggt aag cac gct gct gat    48
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat    96
Glu Glu Lys Leu Tyr Gly Ser Thr Glu Asn Lys Leu Gly Ile Ala Asn
             20                  25                  30

tgg tta aaa gat caa ggt cat gaa cta att act act tct gat aaa gaa   144
Trp Leu Lys Asp Gln Gly His Glu Leu Ile Thr Thr Ser Asp Lys Glu
         35                  40                  45

ggt gaa aca agt gaa ttg gat aaa cat atc cca gat gct gat att atc   192
Gly Glu Thr Ser Glu Leu Asp Lys His Ile Pro Asp Ala Asp Ile Ile
     50                  55                  60

atc acc act cct ttc cat cct gct tat atc act aag gaa aga ctt gac   240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat   288
Lys Ala Lys Asn Leu Lys Leu Val Val Val Ala Gly Val Gly Ser Asp
                 85                  90                  95

cac att gat tta gat tat att aat caa aca ggt aag aaa atc tca gtc   336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc   384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa   432
Met Thr Met Leu Val Leu Val Arg Asn Phe Val Pro Ala His Glu Gln
    130                 135                 140

att att aac cac gat tgg gag gtt gct gct atc gct aag gat gct tac   480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt   528
```

```
Asp Ile Glu Gly Lys Thr Ile Ala Thr Ile Gly Ala Gly Arg Ile Gly
                165                 170                 175

tac agt gtc ttg gaa aga tta ctc cca ttt aat cca aaa gaa tta tta    576
Tyr Ser Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt    624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc    672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat    720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc    768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa    816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca    864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct    912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa    960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                1095
Tyr Val Thr Lys Ala Tyr Gly Lys His Asp Lys Lys
        355                 360                 365
```

<210> 12
<211> 364
<212> PRT
<213> Künstliche Sequenz
<223> Beschreibung der künstlichen Sequenz:C23S und R178S

<400> 12

```
Met Lys Ile Val Leu Val Leu Tyr Asp Ala Gly Lys His Ala Ala Asp
 1               5                  10                  15
Glu Glu Lys Leu Tyr Gly Ser Thr Glu Asn Lys Leu Gly Ile Ala Asn
            20                  25                  30
Trp Leu Lys Asp Gln Gly His Glu Leu Ile Thr Thr Ser Asp Lys Glu
        35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
65                  70                  75                  80
Lys Ala Lys Asn Leu Lys Leu Val Val Val Ala Gly Val Gly Ser Asp
                85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Glu Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Ser Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

<210> 13
<211> 1095
<212> DNA
<213> Künstliche Sequenz

<220>
<223> Beschreibung der künstlichen Sequenz:C23S und R178G

```
<220>
<221> CDS
<222> (1)..(1095)

<400> 13
atg aag att gtc tta gtt ctt tat gat gct ggt aag cac gct gct gat      48
Met Lys Ile Val Leu Val Leu Tyr Asp Ala Gly Lys His Ala Ala Asp
1               5                   10                  15

gaa gaa aaa tta tat ggt tct act gaa aat aaa tta ggt att gct aat      96
Glu Glu Lys Leu Tyr Gly Ser Thr Glu Asn Lys Leu Gly Ile Ala Asn
            20                  25                  30

tgg tta aaa gat caa ggt cat gaa cta att act act tct gat aaa gaa      144
Trp Leu Lys Asp Gln Gly His Glu Leu Ile Thr Thr Ser Asp Lys Glu
        35                  40                  45

ggt gaa aca agt gaa ttg gat aaa cat atc cca gat gct gat att atc      192
Gly Glu Thr Ser Glu Leu Asp Lys His Ile Pro Asp Ala Asp Ile Ile
    50                  55                  60

atc acc act cct ttc cat cct gct tat atc act aag gaa aga ctt gac      240
Ile Thr Thr Pro Phe His Pro Ala Tyr Ile Thr Lys Glu Arg Leu Asp
65                  70                  75                  80

aag gct aag aac tta aaa tta gtc gtt gtc gct ggt gtt ggt tct gat      288
Lys Ala Lys Asn Leu Lys Leu Val Val Val Ala Gly Val Gly Ser Asp
                85                  90                  95

cac att gat tta gat tat att aat caa aca ggt aag aaa atc tca gtc      336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc      384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa      432
Met Thr Met Leu Val Leu Val Arg Asn Phe Val Pro Ala His Glu Gln
    130                 135                 140

att att aac cac gat tgg gag gtt gct gct atc gct aag gat gct tac      480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt      528
Asp Ile Glu Gly Lys Thr Ile Ala Thr Ile Gly Ala Gly Arg Ile Gly
                165                 170                 175

tac gga gtc ttg gaa aga tta ctc cca ttt aat cca aaa gaa tta tta      576
Tyr Gly Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt      624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc      672
Ala Arg Arg Val Glu Asn Ile Glu Glu Leu Val Ala Gln Ala Asp Ile
```

```
        210                 215                 220

gtt aca gtt aat gct cca tta cac gca ggt aca aaa ggt tta att aat     720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc     768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa     816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca     864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct     912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa     960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc     1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa     1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                 1095
Tyr Val Thr Lys Ala Tyr Gly Lys His Asp Lys Lys
        355                 360                 365


<210> 14
<211> 364
<212> PRT
<213> Künstliche Sequenz
<223> Beschreibung der künstlichen Sequenz:C23S und
      R178G

<400> 14
Met Lys Ile Val Leu Val Leu Tyr Asp Ala Gly Lys His Ala Ala Asp
  1               5                  10                  15
Glu Glu Lys Leu Tyr Gly Ser Thr Glu Asn Lys Leu Gly Ile Ala Asn
            20                  25                  30
Trp Leu Lys Asp Gln Gly His Glu Leu Ile Thr Thr Ser Asp Lys Glu
        35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
65                  70                  75                  80
Lys Ala Lys Asn Leu Lys Leu Val Val Val Ala Gly Val Gly Ser Asp
                85                  90                  95
```

```
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Glu Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Gly Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

```
<210> 15
<211> 1095
<212> DNA
<213> Künstliche Sequenz

<220>
<223> Beschreibung der künstlichen Sequenz:C23S und
      K206R

<220>
<221> CDS
<222> (1)..(1095)

<400> 15
atg aag att gtc tta gtt ctt tat gat gct ggt aag cac gct gct gat    48
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat    96
Glu Glu Lys Leu Tyr Gly Ser Thr Glu Asn Lys Leu Gly Ile Ala Asn
            20                  25                  30
```

```
tgg tta aaa gat caa ggt cat gaa cta att act act tct gat aaa gaa      144
Trp Leu Lys Asp Gln Gly His Glu Leu Ile Thr Thr Ser Asp Lys Glu
        35                  40                  45

ggt gaa aca agt gaa ttg gat aaa cat atc cca gat gct gat att atc      192
Gly Glu Thr Ser Glu Leu Asp Lys His Ile Pro Asp Ala Asp Ile Ile
    50                  55                  60

atc acc act cct ttc cat cct gct tat atc act aag gaa aga ctt gac      240
Ile Thr Thr Pro Phe His Pro Ala Tyr Ile Thr Lys Glu Arg Leu Asp
65                  70                  75                  80

aag gct aag aac tta aaa tta gtc gtt gtc gct ggt gtt ggt tct gat      288
Lys Ala Lys Asn Leu Lys Leu Val Val Val Ala Gly Val Gly Ser Asp
                85                  90                  95

cac att gat tta gat tat att aat caa aca ggt aag aaa atc tca gtc      336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc      384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa      432
Met Thr Met Leu Val Leu Val Arg Asn Phe Val Pro Ala His Glu Gln
    130                 135                 140

att att aac cac gat tgg gag gtt gct gct atc gct aag gat gct tac      480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt      528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta      576
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aga gtt ggt      624
Tyr Tyr Asp Tyr Gln Ala Leu Pro Lys Glu Ala Glu Glu Arg Val Gly
        195                 200                 205

gct aga aga gtt gaa aat att gaa gaa tta gtt gct caa gct gat atc      672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat      720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc      768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa      816
Ala Arg Gly Ala Ile Cys Val Ala Glu Asp Val Ala Ala Ala Leu Glu
            260                 265                 270
```

```
tct ggt caa tta aga ggt tac ggt ggt gat gtt tgg ttc cca caa cca    864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct    912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa    960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                1095
Tyr Val Thr Lys Ala Tyr Gly Lys His Asp Lys Lys
        355                 360                 365


<210> 16
<211> 364
<212> PRT
<213> Künstliche Sequenz
<223> Beschreibung der künstlichen Sequenz:C23S und
      K206R

<400> 16
Met Lys Ile Val Leu Val Leu Tyr Asp Ala Gly Lys His Ala Ala Asp
 1               5                  10                  15
Glu Glu Lys Leu Tyr Gly Ser Thr Glu Asn Lys Leu Gly Ile Ala Asn
            20                  25                  30
Trp Leu Lys Asp Gln Gly His Glu Leu Ile Thr Thr Ser Asp Lys Glu
        35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
65                  70                  75                  80
Lys Ala Lys Asn Leu Lys Leu Val Val Val Ala Gly Val Gly Ser Asp
                85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Glu Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Arg Val Gly
```

```
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

```
<210> 17
<211> 1095
<212> DNA
<213> Künstliche Sequenz

<220>
<223> Beschreibung der künstlichen Sequenz:C23S und
      F285Y

<220>
<221> CDS
<222> (1)..(1095)

<400> 17
atg aag att gtc tta gtt ctt tat gat gct ggt aag cac gct gct gat      48
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat      96
Glu Glu Lys Leu Tyr Gly Ser Thr Glu Asn Lys Leu Gly Ile Ala Asn
            20                  25                  30

tgg tta aaa gat caa ggt cat gaa cta att act act tct gat aaa gaa     144
Trp Leu Lys Asp Gln Gly His Glu Leu Ile Thr Thr Ser Asp Lys Glu
        35                  40                  45

ggt gaa aca agt gaa ttg gat aaa cat atc cca gat gct gat att atc     192
Gly Glu Thr Ser Glu Leu Asp Lys His Ile Pro Asp Ala Asp Ile Ile
    50                  55                  60

atc acc act cct ttc cat cct gct tat atc act aag gaa aga ctt gac     240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat     288
```

```
Lys Ala Lys Asn Leu Lys Leu Val Val Val Ala Gly Val Gly Ser Asp
                 85                  90                  95

cac att gat tta gat tat att aat caa aca ggt aag aaa atc tca gtc     336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc     384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa     432
Met Thr Met Leu Val Leu Val Arg Asn Phe Val Pro Ala His Glu Gln
    130                 135                 140

att att aac cac gat tgg gag gtt gct gct atc gct aag gat gct tac     480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt     528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta     576
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt     624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc     672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat     720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc     768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa     816
Ala Arg Gly Ala Ile Cys Val Ala Glu Asp Val Ala Ala Ala Leu Glu
            260                 265                 270

tct ggt caa tta aga ggt tac ggt ggt gat gtt tgg tac cca caa cca     864
Ser Gly Gln Leu Arg Gly Tyr Gly Gly Asp Val Trp Tyr Pro Gln Pro
        275                 280                 285

gct cca aag gat cac cca tgg aga gat atg aga aat aaa tat ggt gct     912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa     960
Gly Asn Ala Met Thr Pro His Tyr Ser Gly Thr Thr Leu Asp Ala Gln
305                 310                 315                 320
```

```
aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                1095
Tyr Val Thr Lys Ala Tyr Gly Lys His Asp Lys Lys
        355                 360                 365


<210> 18
<211> 364
<212> PRT
<213> Künstliche Sequenz
<223> Beschreibung der künstlichen Sequenz:C23S und
      F285Y

<400> 18
Met Lys Ile Val Leu Val Leu Tyr Asp Ala Gly Lys His Ala Ala Asp
  1               5                  10                  15
Glu Glu Lys Leu Tyr Gly Ser Thr Glu Asn Lys Leu Gly Ile Ala Asn
            20                  25                  30
Trp Leu Lys Asp Gln Gly His Glu Leu Ile Thr Thr Ser Asp Lys Glu
        35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
 65                  70                  75                  80
Lys Ala Lys Asn Leu Lys Leu Val Val Val Ala Gly Val Gly Ser Asp
                 85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Glu Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Tyr Pro Gln Pro
        275                 280                 285
Ala Pro Lys Asp His Pro Trp Arg Asp Met Arg Asn Lys Tyr Gly Ala
    290                 295                 300
```

```
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

<210> 19
<211> 1095
<212> DNA
<213> Künstliche Sequenz

<220>
<223> Beschreibung der künstlichen Sequenz:C23S und
      F285S

<220>
<221> CDS
<222> (1)..(1095)

<400> 19

```
atg aag att gtc tta gtt ctt tat gat gct ggt aag cac gct gct gat      48
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat      96
Glu Glu Lys Leu Tyr Gly Ser Thr Glu Asn Lys Leu Gly Ile Ala Asn
             20                  25                  30

tgg tta aaa gat caa ggt cat gaa cta att act act tct gat aaa gaa     144
Trp Leu Lys Asp Gln Gly His Glu Leu Ile Thr Thr Ser Asp Lys Glu
         35                  40                  45

ggt gaa aca agt gaa ttg gat aaa cat atc cca gat gct gat att atc     192
Gly Glu Thr Ser Glu Leu Asp Lys His Ile Pro Asp Ala Asp Ile Ile
     50                  55                  60

atc acc act cct ttc cat cct gct tat atc act aag gaa aga ctt gac     240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat     288
Lys Ala Lys Asn Leu Lys Leu Val Val Val Ala Gly Val Gly Ser Asp
                 85                  90                  95

cac att gat tta gat tat att aat caa aca ggt aag aaa atc tca gtc     336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc     384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa     432
Met Thr Met Leu Val Leu Val Arg Asn Phe Val Pro Ala His Glu Gln
```

```
        130                 135                 140

att att aac cac gat tgg gag gtt gct gct atc gct aag gat gct tac    480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt    528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta    576
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt    624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc    672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat    720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc    768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa    816
Ala Arg Gly Ala Ile Cys Val Ala Glu Asp Val Ala Ala Ala Leu Glu
            260                 265                 270

tct ggt caa tta aga ggt tac ggt ggt gat gtt tgg tcc cca caa cca    864
Ser Gly Gln Leu Arg Gly Tyr Gly Gly Asp Val Trp Ser Pro Gln Pro
        275                 280                 285

gct cca aag gat cac cca tgg aga gat atg aga aat aaa tat ggt gct    912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa    960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                1095
Tyr Val Thr Lys Ala Tyr Gly Lys His Asp Lys Lys
        355                 360                 365
```

<210> 20
<211> 364
<212> PRT
<213> Künstliche Sequenz
<223> Beschreibung der künstlichen Sequenz:C23S und F285S

<400> 20
Met Lys Ile Val Leu Val Leu Tyr Asp Ala Gly Lys His Ala Ala Asp
1 5 10 15
Glu Glu Lys Leu Tyr Gly Ser Thr Glu Asn Lys Leu Gly Ile Ala Asn
20 25 30
Trp Leu Lys Asp Gln Gly His Glu Leu Ile Thr Thr Ser Asp Lys Glu
35 40 45
Gly Glu Thr Ser Glu Leu Asp Lys His Ile Pro Asp Ala Asp Ile Ile
50 55 60
Ile Thr Thr Pro Phe His Pro Ala Tyr Ile Thr Lys Glu Arg Leu Asp
65 70 75 80
Lys Ala Lys Asn Leu Lys Leu Val Val Val Ala Gly Val Gly Ser Asp
85 90 95
His Ile Asp Leu Asp Tyr Ile Asn Gln Thr Gly Lys Lys Ile Ser Val
100 105 110
Leu Glu Val Thr Gly Ser Asn Val Val Ser Val Ala Glu His Val Val
115 120 125
Met Thr Met Leu Val Leu Val Arg Asn Phe Val Pro Ala His Glu Gln
130 135 140
Ile Ile Asn His Asp Trp Glu Val Ala Ala Ile Ala Lys Asp Ala Tyr
145 150 155 160
Asp Ile Glu Gly Lys Thr Ile Ala Thr Ile Gly Ala Gly Arg Ile Gly
165 170 175
Tyr Arg Val Leu Glu Arg Leu Leu Pro Phe Asn Pro Lys Glu Leu Leu
180 185 190
Tyr Tyr Asp Tyr Gln Ala Leu Pro Lys Glu Ala Glu Glu Lys Val Gly
195 200 205
Ala Arg Arg Val Glu Asn Ile Glu Glu Leu Val Ala Gln Ala Asp Ile
210 215 220
Val Thr Val Asn Ala Pro Leu His Ala Gly Thr Lys Gly Leu Ile Asn
225 230 235 240
Lys Glu Leu Leu Ser Lys Phe Lys Lys Gly Ala Trp Leu Val Asn Thr
245 250 255
Ala Arg Gly Ala Ile Cys Val Ala Glu Asp Val Ala Ala Ala Leu Glu
260 265 270
Ser Gly Gln Leu Arg Gly Tyr Gly Gly Asp Val Trp Ser Pro Gln Pro
275 280 285
Ala Pro Lys Asp His Pro Trp Arg Asp Met Arg Asn Lys Tyr Gly Ala
290 295 300
Gly Asn Ala Met Thr Pro His Tyr Ser Gly Thr Thr Leu Asp Ala Gln
305 310 315 320
Thr Arg Tyr Ala Glu Gly Thr Lys Asn Ile Leu Glu Ser Phe Phe Thr
325 330 335
Gly Lys Phe Asp Tyr Arg Pro Gln Asp Ile Ile Leu Leu Asn Gly Glu
340 345 350
Tyr Val Thr Lys Ala Tyr Gly Lys His Asp Lys Lys
355 360

<210> 21
<211> 1095

```
<212> DNA
<213> Künstliche Sequenz

<220>
<223> Beschreibung der künstlichen Sequenz:C23S und
      T315N

<220>
<221> CDS
<222> (1)..(1095)

<400> 21
atg aag att gtc tta gtt ctt tat gat gct ggt aag cac gct gct gat     48
Met Lys Ile Val Leu Val Leu Tyr Asp Ala Gly Lys His Ala Ala Asp
  1               5                  10                  15

gaa gaa aaa tta tat ggt tct act gaa aat aaa tta ggt att gct aat     96
Glu Glu Lys Leu Tyr Gly Ser Thr Glu Asn Lys Leu Gly Ile Ala Asn
             20                  25                  30

tgg tta aaa gat caa ggt cat gaa cta att act act tct gat aaa gaa    144
Trp Leu Lys Asp Gln Gly His Glu Leu Ile Thr Thr Ser Asp Lys Glu
         35                  40                  45

ggt gaa aca agt gaa ttg gat aaa cat atc cca gat gct gat att atc    192
Gly Glu Thr Ser Glu Leu Asp Lys His Ile Pro Asp Ala Asp Ile Ile
     50                  55                  60

atc acc act cct ttc cat cct gct tat atc act aag gaa aga ctt gac    240
Ile Thr Thr Pro Phe His Pro Ala Tyr Ile Thr Lys Glu Arg Leu Asp
 65                  70                  75                  80

aag gct aag aac tta aaa tta gtc gtt gtc gct ggt gtt ggt tct gat    288
Lys Ala Lys Asn Leu Lys Leu Val Val Val Ala Gly Val Gly Ser Asp
                 85                  90                  95

cac att gat tta gat tat att aat caa aca ggt aag aaa atc tca gtc    336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc    384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa    432
Met Thr Met Leu Val Leu Val Arg Asn Phe Val Pro Ala His Glu Gln
    130                 135                 140

att att aac cac gat tgg gag gtt gct gct atc gct aag gat gct tac    480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt    528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta    576
Tyr Arg Val Leu Glu Arg Leu Leu Pro Phe Asn Pro Lys Glu Leu Leu
            180                 185                 190
```

```
tac tac gat tat caa gct tta cca aaa gaa gct gaa gaa aaa gtt ggt     624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc     672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat     720
Val Thr Val Asn Ala Pro Leu His Ala Gly Thr Lys Gly Leu Ile Asn
225                 230                 235                 240

aag gaa tta tta tct aaa ttt aaa aaa ggt gct tgg tta gtc aat acc     768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa     816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca     864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct     912
Ala Pro Lys Asp His Pro Trp Arg Asp Met Arg Asn Lys Tyr Gly Ala
    290                 295                 300

ggt aat gcc atg act cct cac tac tct ggt aat act tta gac gct caa     960
Gly Asn Ala Met Thr Pro His Tyr Ser Gly Asn Thr Leu Asp Ala Gln
305                 310                 315                 320

aca aga tac gct gaa ggt act aaa aat att ttg gaa tca ttc ttt acc     1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa     1056
Gly Lys Phe Asp Tyr Arg Pro Gln Asp Ile Ile Leu Leu Asn Gly Glu
            340                 345                 350

tac gtt act aaa gct tac ggt aaa cac gat aag aaa taa                 1095
Tyr Val Thr Lys Ala Tyr Gly Lys His Asp Lys Lys
        355                 360                 365


<210> 22
<211> 364
<212> PRT
<213> Künstliche Sequenz
<223> Beschreibung der künstlichen Sequenz:C23S und
      T315N

<400> 22
Met Lys Ile Val Leu Val Leu Tyr Asp Ala Gly Lys His Ala Ala Asp
  1               5                  10                  15
Glu Glu Lys Leu Tyr Gly Ser Thr Glu Asn Lys Leu Gly Ile Ala Asn
            20                  25                  30
Trp Leu Lys Asp Gln Gly His Glu Leu Ile Thr Thr Ser Asp Lys Glu
```

```
                35                  40                  45
Gly Glu Thr Ser Glu Leu Asp Lys His Ile Pro Asp Ala Asp Ile Ile
    50                  55                  60
Ile Thr Thr Pro Phe His Pro Ala Tyr Ile Thr Lys Glu Arg Leu Asp
65                  70                  75                  80
Lys Ala Lys Asn Leu Lys Leu Val Val Val Ala Gly Val Gly Ser Asp
                85                  90                  95
His Ile Asp Leu Asp Tyr Ile Asn Gln Thr Gly Lys Lys Ile Ser Val
            100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
Ile Ile Asn His Asp Trp Glu Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Asn Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Lys Ala Tyr Gly Lys His Asp Lys Lys
        355                 360
```

<210> 23
<211> 1095
<212> DNA
<213> Künstliche Sequenz

<220>
<223> Beschreibung der künstlichen Sequenz:C23S und K356E

<220>
<221> CDS
<222> (1)..(1095)

<400> 23

```
atg aag att gtc tta gtt ctt tat gat gct ggt aag cac gct gct gat    48
```

```
Met Lys Ile Val Leu Val Leu Tyr Asp Ala Gly Lys His Ala Ala Asp
1               5                   10                  15

gaa gaa aaa tta tat ggt tct act gaa aat aaa tta ggt att gct aat      96
Glu Glu Lys Leu Tyr Gly Ser Thr Glu Asn Lys Leu Gly Ile Ala Asn
            20                  25                  30

tgg tta aaa gat caa ggt cat gaa cta att act act tct gat aaa gaa      144
Trp Leu Lys Asp Gln Gly His Glu Leu Ile Thr Thr Ser Asp Lys Glu
        35                  40                  45

ggt gaa aca agt gaa ttg gat aaa cat atc cca gat gct gat att atc      192
Gly Glu Thr Ser Glu Leu Asp Lys His Ile Pro Asp Ala Asp Ile Ile
    50                  55                  60

atc acc act cct ttc cat cct gct tat atc act aag gaa aga ctt gac      240
Ile Thr Thr Pro Phe His Pro Ala Tyr Ile Thr Lys Glu Arg Leu Asp
65                  70                  75                  80

aag gct aag aac tta aaa tta gtc gtt gtc gct ggt gtt ggt tct gat      288
Lys Ala Lys Asn Leu Lys Leu Val Val Val Ala Gly Val Gly Ser Asp
                85                  90                  95

cac att gat tta gat tat att aat caa aca ggt aag aaa atc tca gtc      336
His Ile Asp Leu Asp Tyr Ile Asn Gln Thr Gly Lys Lys Ile Ser Val
            100                 105                 110

ctg gaa gtt aca ggt tct aat gtt gtc tct gtt gct gaa cac gtt gtc      384
Leu Glu Val Thr Gly Ser Asn Val Val Ser Val Ala Glu His Val Val
        115                 120                 125

atg acc atg ctt gtc ttg gtt aga aat ttc gtt cca gca cat gaa caa      432
Met Thr Met Leu Val Leu Val Arg Asn Phe Val Pro Ala His Glu Gln
    130                 135                 140

att att aac cac gat tgg gag gtt gct gct atc gct aag gat gct tac      480
Ile Ile Asn His Asp Trp Glu Val Ala Ala Ile Ala Lys Asp Ala Tyr
145                 150                 155                 160

gat atc gaa ggt aaa act atc gct acc att ggt gct ggt aga att ggt      528
Asp Ile Glu Gly Lys Thr Ile Ala Thr Ile Gly Ala Gly Arg Ile Gly
                165                 170                 175

tac aga gtc ttg gaa aga tta ctc cca ttt aat cca aaa gaa tta tta      576
Tyr Arg Val Leu Glu Arg Leu Leu Pro Phe Asn Pro Lys Glu Leu Leu
            180                 185                 190

tac tac gat tat caa gct tta cca aaa gaa gct gaa gaa aaa gtt ggt      624
Tyr Tyr Asp Tyr Gln Ala Leu Pro Lys Glu Ala Glu Glu Lys Val Gly
        195                 200                 205

gct aga aga gtt gaa aat att gaa gaa tta gtt gct caa gct gat atc      672
Ala Arg Arg Val Glu Asn Ile Glu Glu Leu Val Ala Gln Ala Asp Ile
    210                 215                 220

gtt aca gtt aat gct cca tta cac gca ggt aca aaa ggt tta att aat      720
Val Thr Val Asn Ala Pro Leu His Ala Gly Thr Lys Gly Leu Ile Asn
225                 230                 235                 240
```

```
aag gaa tta tta tct aaa ttt aaa aaa ggt gct tgg tta gtc aat acc     768
Lys Glu Leu Leu Ser Lys Phe Lys Lys Gly Ala Trp Leu Val Asn Thr
                245                 250                 255

gca aga ggt gct att tgt gtt gct gaa gat gtt gca gca gct tta gaa     816
Ala Arg Gly Ala Ile Cys Val Ala Glu Asp Val Ala Ala Ala Leu Glu
            260                 265                 270

tct ggt caa tta aga ggt tac ggt ggt gat gtt tgg ttc cca caa cca     864
Ser Gly Gln Leu Arg Gly Tyr Gly Gly Asp Val Trp Phe Pro Gln Pro
        275                 280                 285

gct cca aag gat cac cca tgg aga gat atg aga aat aaa tat ggt gct     912
Ala Pro Lys Asp His Pro Trp Arg Asp Met Arg Asn Lys Tyr Gly Ala
    290                 295                 300

ggt aat gcc atg act cct cac tac tct ggt act act tta gac gct caa     960
Gly Asn Ala Met Thr Pro His Tyr Ser Gly Thr Thr Leu Asp Ala Gln
305                 310                 315                 320

aca aga tac gct gaa ggt act aaa aat att ttg gaa tca ttc ttt acc    1008
Thr Arg Tyr Ala Glu Gly Thr Lys Asn Ile Leu Glu Ser Phe Phe Thr
                325                 330                 335

ggt aaa ttt gat tac aga cca caa gat att atc tta tta aat ggt gaa    1056
Gly Lys Phe Asp Tyr Arg Pro Gln Asp Ile Ile Leu Leu Asn Gly Glu
            340                 345                 350

tac gtt act gaa gct tac ggt aaa cac gat aag aaa taa                1095
Tyr Val Thr Glu Ala Tyr Gly Lys His Asp Lys Lys
        355                 360                 365


<210> 24
<211> 364
<212> PRT
<213> Künstliche Sequenz
<223> Beschreibung der künstlichen Sequenz:C23S und
      K356E

<400> 24
Met Lys Ile Val Leu Val Leu Tyr Asp Ala Gly Lys His Ala Ala Asp
  1               5                  10                  15
Glu Glu Lys Leu Tyr Gly Ser Thr Glu Asn Lys Leu Gly Ile Ala Asn
                 20                  25                  30
Trp Leu Lys Asp Gln Gly His Glu Leu Ile Thr Thr Ser Asp Lys Glu
         35                  40                  45
Gly Glu Thr Ser Glu Leu Asp Lys His Ile Pro Asp Ala Asp Ile Ile
     50                  55                  60
Ile Thr Thr Pro Phe His Pro Ala Tyr Ile Thr Lys Glu Arg Leu Asp
 65                  70                  75                  80
Lys Ala Lys Asn Leu Lys Leu Val Val Val Ala Gly Val Gly Ser Asp
                 85                  90                  95
His Ile Asp Leu Asp Tyr Ile Asn Gln Thr Gly Lys Lys Ile Ser Val
                100                 105                 110
Leu Glu Val Thr Gly Ser Asn Val Val Ser Val Ala Glu His Val Val
        115                 120                 125
Met Thr Met Leu Val Leu Val Arg Asn Phe Val Pro Ala His Glu Gln
    130                 135                 140
```

```
Ile Ile Asn His Asp Trp Glu Val Ala Ala Ile Ala Lys Asp Ala Tyr
145                 150                 155                 160
Asp Ile Glu Gly Lys Thr Ile Ala Thr Ile Gly Ala Gly Arg Ile Gly
                165                 170                 175
Tyr Arg Val Leu Glu Arg Leu Leu Pro Phe Asn Pro Lys Glu Leu Leu
            180                 185                 190
Tyr Tyr Asp Tyr Gln Ala Leu Pro Lys Glu Ala Glu Glu Lys Val Gly
        195                 200                 205
Ala Arg Arg Val Glu Asn Ile Glu Glu Leu Val Ala Gln Ala Asp Ile
    210                 215                 220
Val Thr Val Asn Ala Pro Leu His Ala Gly Thr Lys Gly Leu Ile Asn
225                 230                 235                 240
Lys Glu Leu Leu Ser Lys Phe Lys Lys Gly Ala Trp Leu Val Asn Thr
                245                 250                 255
Ala Arg Gly Ala Ile Cys Val Ala Glu Asp Val Ala Ala Ala Leu Glu
            260                 265                 270
Ser Gly Gln Leu Arg Gly Tyr Gly Gly Asp Val Trp Phe Pro Gln Pro
        275                 280                 285
Ala Pro Lys Asp His Pro Trp Arg Asp Met Arg Asn Lys Tyr Gly Ala
    290                 295                 300
Gly Asn Ala Met Thr Pro His Tyr Ser Gly Thr Thr Leu Asp Ala Gln
305                 310                 315                 320
Thr Arg Tyr Ala Glu Gly Thr Lys Asn Ile Leu Glu Ser Phe Phe Thr
                325                 330                 335
Gly Lys Phe Asp Tyr Arg Pro Gln Asp Ile Ile Leu Leu Asn Gly Glu
            340                 345                 350
Tyr Val Thr Glu Ala Tyr Gly Lys His Asp Lys Lys
        355                 360
```

<210> 25
<211> 20
<212> DNA
<213> Künstliche Sequenz

<220>
<223> Beschreibung der künstlichen Sequenz:PBTACF1

<400> 25
tgcctggcag ttccctactc 20

<210> 26
<211> 20
<212> DNA
<213> Künstliche Sequenz

<220>
<223> Beschreibung der künstlichen Sequenz:PBTACF2

<400> 26
cgtttcactt ctgagttcgg 20

<210> 27
<211> 20
<212> DNA
<213> Künstliche Sequenz

<220>
<223> Beschreibung der künstlichen Sequenz:PBTACR1

<400> 27
ggtatggctg tgcaggtcgt 20

<210> 28
<211> 20
<212> DNA
<213> Künstliche Sequenz

<220>
<223> Beschreibung der künstlichen Sequenz:PBTACR2

<400> 28
cgacatcata acggttctgg 20

<210> 29
<211> 20
<212> DNA
<213> Künstliche Sequenz

<220>
<223> Beschreibung der künstlichen Sequenz:PBTACR3

<400> 29
tcatcggctc gtataatgtg 20

<210> 30
<211> 33
<212> DNA
<213> Künstliche Sequenz

<220>
<223> Beschreibung der künstlichen Sequenz:Primer F285S-F1

<400> 30
ggtgatgttt ggtccccaca accagctcca aag 33

<210> 31
<211> 32
<212> DNA
<213> Künstliche Sequenz

<220>
<223> Beschreibung der künstlichen Sequenz:Primer F285S-F2

<400> 31
ggagctggtt gtggggacca aacatcaccg ta 32

<210> 32

<211> 364
<212> PRT
<213> Künstliche Sequenz

<220>
<223> Beschreibung der künstlichen Sequenz:DM-3bE10

<400> 32

```
Met Lys Ile Val Leu Val Leu Tyr Asp Ala Gly Lys His Ala Ala Asp
  1               5                  10                  15

Glu Glu Lys Leu Tyr Gly Ser Thr Glu Asn Lys Leu Gly Ile Ala Asn
             20                  25                  30

Trp Leu Lys Asp Gln Gly His Glu Leu Ile Thr Thr Ser Asp Lys Glu
         35                  40                  45

Gly Glu Thr Ser Glu Leu Asp Lys His Ile Pro Asp Ala Asp Ile Ile
     50                  55                  60

Ile Thr Thr Pro Phe His Pro Ala Tyr Ile Thr Lys Glu Arg Leu Asp
 65                  70                  75                  80

Lys Ala Lys Asn Leu Lys Leu Val Val Val Ala Gly Val Gly Ser Asp
                 85                  90                  95

His Ile Asp Leu Asp Tyr Ile Asn Gln Thr Gly Lys Lys Ile Ser Val
            100                 105                 110

Leu Glu Val Thr Gly Ser Asn Val Val Ser Val Ala Glu His Val Val
        115                 120                 125

Met Thr Met Leu Val Leu Val Arg Asn Phe Val Pro Ala His Glu Gln
    130                 135                 140

Ile Ile Asn His Asp Trp Glu Val Ala Ala Ile Ala Lys Asp Ala Tyr
145                 150                 155                 160

Asp Ile Glu Gly Lys Thr Ile Ala Thr Ile Gly Ala Gly Arg Ile Gly
                165                 170                 175

Tyr Arg Val Leu Glu Arg Leu Leu Pro Phe Asn Pro Lys Glu Leu Leu
            180                 185                 190

Tyr Tyr Asp Tyr Gln Ala Leu Pro Lys Glu Ala Glu Glu Arg Val Gly
        195                 200                 205

Ala Arg Arg Val Glu Asn Ile Glu Glu Leu Val Ala Gln Ala Asp Ile
    210                 215                 220

Val Thr Val Asn Ala Pro Leu His Ala Gly Thr Lys Gly Leu Ile Asn
225                 230                 235                 240

Lys Glu Leu Leu Ser Lys Phe Lys Lys Gly Ala Trp Leu Val Asn Thr
                245                 250                 255

Ala Arg Gly Ala Ile Ala Val Ala Glu Asp Val Ala Ala Ala Leu Glu
            260                 265                 270
```

Ser Gly Gln Leu Arg Gly Tyr Gly Gly Asp Val Trp Phe Pro Gln Pro
275 280 285

Ala Pro Lys Asp His Pro Trp Arg Asp Met Arg Asn Lys Tyr Gly Ala
290 295 300

Gly Asn Ala Met Thr Pro His Tyr Ser Gly Asn Thr Leu Asp Ala Gln
305 310 315 320

Thr Arg Tyr Ala Glu Gly Thr Lys Asn Ile Leu Glu Ser Phe Phe Thr
325 330 335

Gly Lys Phe Asp Tyr Arg Pro Gln Asp Ile Ile Leu Leu Asn Gly Glu
340 345 350

Tyr Val Thr Glu Ala Tyr Gly Lys His Asp Lys Lys
355 360

<210> 33
<211> 364
<212> PRT
<213> Künstliche Sequenz

<220>
<223> Beschreibung der künstlichen Sequenz:DM-2kA6

<400> 33
Met Lys Ile Val Leu Val Leu Tyr Asp Ala Gly Lys His Ala Ala Asp
1 5 10 15

Glu Asp Lys Leu Tyr Gly Ser Thr Glu Asn Lys Leu Gly Ile Ala Asn
20 25 30

Trp Leu Arg Asp Gln Gly His Glu Leu Ile Thr Thr Ser Asp Lys Glu
35 40 45

Gly Glu Thr Ser Glu Leu Asp Lys His Ile Pro Asp Ala Asp Ile Ile
50 55 60

Ile Thr Thr Pro Phe His Pro Ala Tyr Ile Thr Lys Glu Arg Leu Asp
65 70 75 80

Lys Ala Lys Asn Leu Lys Leu Val Val Val Ala Gly Val Gly Ser Asp
85 90 95

His Ile Asp Leu Asp Tyr Ile Asn Gln Thr Gly Lys Lys Ile Ser Val
100 105 110

Leu Glu Val Thr Gly Ser Asn Val Val Ser Val Ala Glu His Val Val
115 120 125

Met Thr Met Leu Val Leu Val Arg Asn Phe Val Pro Ala His Glu Gln
130 135 140

Ile Ile Asn His Asp Trp Glu Val Ala Ala Ile Ala Lys Asp Ala Tyr
145 150 155 160

Asp Ile Glu Gly Lys Thr Ile Ala Thr Ile Gly Ala Gly Arg Ile Gly

```
                165                 170                 175

Tyr Ser Val Leu Glu Arg Leu Leu Pro Phe Asn Pro Lys Glu Leu Leu
            180                 185                 190

Tyr Tyr Asp Tyr Gln Ala Leu Pro Lys Glu Ala Glu Glu Lys Val Gly
        195                 200                 205

Ala Arg Arg Val Glu Asn Ile Glu Glu Leu Val Ala Gln Ala Asp Ile
    210                 215                 220

Val Thr Val Asn Ala Pro Leu His Ala Gly Thr Lys Gly Leu Ile Asn
225                 230                 235                 240

Lys Glu Leu Leu Ser Lys Phe Lys Lys Gly Ala Trp Leu Val Asn Thr
                245                 250                 255

Ala Arg Gly Ala Ile Ala Val Ala Glu Asp Val Ala Ala Ala Leu Glu
            260                 265                 270

Ser Gly Gln Leu Arg Gly Tyr Gly Gly Asp Val Trp Phe Pro Gln Pro
        275                 280                 285

Ala Pro Lys Asp His Pro Trp Arg Asp Met Arg Asn Lys Tyr Gly Ala
    290                 295                 300

Gly Asn Ala Met Thr Pro His Tyr Ser Gly Thr Thr Leu Asp Ala Gln
305                 310                 315                 320

Thr Arg Tyr Ala Glu Gly Thr Lys Asn Ile Leu Glu Ser Phe Phe Thr
                325                 330                 335

Gly Lys Phe Asp Tyr Arg Pro Gln Asp Ile Ile Leu Leu Asn Gly Glu
            340                 345                 350

Tyr Val Thr Lys Ala Tyr Gly Lys His Asp Lys Lys
        355                 360
```

<210> 34
<211> 364
<212> PRT
<213> Künstliche Sequenz

<220>
<223> Beschreibung der künstlichen Sequenz:DM-2hG12

<400> 34

```
Met Lys Ile Val Leu Val Leu Tyr Asp Ala Gly Lys His Ala Ala Asp
  1               5                  10                  15

Glu Asp Lys Leu Tyr Gly Ser Thr Glu Asn Lys Leu Gly Ile Ala Asn
             20                  25                  30

Trp Leu Arg Asp Gln Gly His Glu Leu Ile Thr Thr Ser Asp Lys Glu
         35                  40                  45

Gly Glu Thr Ser Glu Leu Asp Lys His Ile Pro Asp Ala Asp Ile Ile
     50                  55                  60
```

```
Ile Thr Thr Pro Phe His Pro Ala Tyr Ile Thr Lys Glu Arg Leu Asp
 65                 70                  75                  80

Lys Ala Lys Asn Leu Lys Leu Val Val Val Ala Gly Val Gly Ser Asp
                85                  90                  95

His Ile Asp Leu Asp Tyr Ile Asn Gln Thr Gly Lys Lys Ile Ser Val
            100                 105                 110

Leu Glu Val Thr Gly Ser Asn Val Val Ser Val Ala Glu His Val Val
        115                 120                 125

Met Thr Met Leu Val Leu Val Arg Asn Phe Val Pro Ala His Glu Gln
    130                 135                 140

Ile Ile Asn His Asp Trp Asp Val Ala Ala Ile Ala Lys Asp Ala Tyr
145                 150                 155                 160

Asp Ile Glu Gly Lys Thr Ile Ala Thr Ile Gly Ala Gly Arg Ile Gly
                165                 170                 175

Tyr Ser Val Leu Glu Arg Leu Leu Pro Phe Asn Pro Lys Glu Leu Leu
            180                 185                 190

Tyr Tyr Asp Tyr Gln Ala Leu Pro Lys Glu Ala Glu Glu Lys Val Gly
        195                 200                 205

Ala Arg Arg Val Glu Asn Ile Glu Glu Leu Val Ala Gln Ala Asp Ile
    210                 215                 220

Val Thr Val Asn Ala Pro Leu His Ala Gly Thr Lys Gly Leu Ile Asn
225                 230                 235                 240

Lys Glu Leu Leu Ser Lys Phe Lys Lys Gly Ala Trp Leu Val Asn Thr
                245                 250                 255

Ala Arg Gly Ala Ile Ala Val Ala Glu Asp Val Ala Ala Ala Leu Glu
            260                 265                 270

Ser Gly Gln Leu Arg Gly Tyr Gly Gly Asp Val Trp Tyr Pro Gln Pro
        275                 280                 285

Ala Pro Lys Asp His Pro Trp Arg Asp Met Arg Asn Lys Tyr Gly Ala
    290                 295                 300

Gly Asn Ala Met Thr Pro His Tyr Ser Gly Thr Thr Leu Asp Ala Gln
305                 310                 315                 320

Thr Arg Tyr Ala Glu Gly Thr Lys Asn Ile Leu Glu Ser Phe Phe Thr
                325                 330                 335

Gly Lys Phe Asp Tyr Arg Pro Gln Asp Ile Ile Leu Leu Asn Gly Glu
            340                 345                 350

Tyr Val Thr Lys Ala Tyr Gly Lys His Asp Lys Lys
        355                 360
```

<210> 35
<211> 364
<212> PRT
<213> Künstliche Sequenz

<220>
<223> Beschreibung der künstlichen Sequenz:SM-1kA2

<400> 35
Met Lys Ile Val Leu Val Leu Tyr Asp Ala Gly Lys His Ala Ala Asp
1               5                   10                  15

Glu Glu Lys Leu Tyr Gly Ser Thr Glu Asn Lys Leu Gly Ile Ala Asn
            20                  25                  30

Trp Leu Lys Asp Gln Gly His Glu Leu Ile Thr Thr Ser Asp Lys Glu
        35                  40                  45

Gly Glu Thr Ser Glu Leu Asp Lys His Ile Pro Asp Ala Asp Ile Ile
    50                  55                  60

Ile Thr Thr Pro Phe His Pro Ala Tyr Ile Thr Lys Glu Arg Leu Asp
65                  70                  75                  80

Lys Ala Lys Asn Leu Lys Leu Val Val Val Ala Gly Val Gly Ser Asp
                85                  90                  95

His Ile Asp Leu Asp Tyr Ile Asn Gln Thr Gly Lys Lys Ile Ser Val
            100                 105                 110

Leu Glu Val Thr Gly Ser Asn Val Val Ser Val Ala Glu His Val Val
        115                 120                 125

Met Thr Met Leu Val Leu Val Arg Asn Phe Val Pro Ala His Glu Gln
    130                 135                 140

Ile Ile Asn His Asp Trp Glu Val Ala Ala Ile Ala Lys Asp Ala Tyr
145                 150                 155                 160

Asp Ile Glu Gly Lys Thr Ile Ala Thr Ile Gly Ala Gly Arg Ile Gly
                165                 170                 175

Tyr Ser Val Leu Glu Arg Leu Leu Pro Phe Asn Pro Lys Glu Leu Leu
            180                 185                 190

Tyr Tyr Asp Tyr Gln Ala Leu Pro Lys Glu Ala Glu Glu Lys Val Gly
        195                 200                 205

Ala Arg Arg Val Glu Asn Ile Glu Glu Leu Val Ala Gln Ala Asp Ile
    210                 215                 220

Val Thr Val Asn Ala Pro Leu His Ala Gly Thr Lys Gly Leu Ile Asn
225                 230                 235                 240

Lys Glu Leu Leu Ser Lys Phe Lys Lys Gly Ala Trp Leu Val Asn Thr
                245                 250                 255

Ala Arg Gly Ala Ile Cys Val Ala Glu Asp Val Ala Ala Ala Leu Glu
            260                 265                 270

```
Ser Gly Gln Leu Arg Gly Tyr Gly Gly Asp Val Trp Phe Pro Gln Pro
        275                 280                 285

Ala Pro Lys Asp His Pro Trp Arg Asp Met Arg Asn Lys Tyr Gly Ala
    290                 295                 300

Gly Asn Ala Met Thr Pro His Tyr Ser Gly Thr Thr Leu Asp Ala Gln
305                 310                 315                 320

Thr Arg Tyr Ala Glu Gly Thr Lys Asn Ile Leu Glu Ser Phe Phe Thr
                325                 330                 335

Gly Lys Phe Asp Tyr Arg Pro Gln Asp Ile Ile Leu Leu Asn Gly Glu
            340                 345                 350

Tyr Val Thr Lys Ala Tyr Gly Lys His Asp Lys Lys
        355                 360


<210> 36
<211> 364
<212> PRT
<213> Künstliche Sequenz

<220>
<223> Beschreibung der künstlichen Sequenz:SM-1eA6

<400> 36
Met Lys Ile Val Leu Val Leu Tyr Asp Ala Gly Lys His Ala Ala Asp
  1               5                  10                  15

Glu Glu Lys Leu Tyr Gly Ser Thr Glu Asn Lys Leu Gly Ile Ala Asn
             20                  25                  30

Trp Leu Lys Asp Gln Gly His Glu Leu Ile Thr Thr Ser Asp Lys Glu
         35                  40                  45

Gly Glu Thr Ser Glu Leu Asp Lys His Ile Pro Asp Ala Asp Ile Ile
     50                  55                  60

Ile Thr Thr Pro Phe His Pro Ala Tyr Ile Thr Lys Glu Arg Leu Asp
 65                  70                  75                  80

Lys Ala Lys Asn Leu Lys Leu Val Val Val Ala Gly Val Gly Ser Asp
                 85                  90                  95

His Ile Asp Leu Asp Tyr Ile Asn Gln Thr Gly Lys Lys Ile Ser Val
            100                 105                 110

Leu Glu Val Thr Gly Ser Asn Val Val Ser Val Ala Glu His Val Val
        115                 120                 125

Met Thr Met Leu Val Leu Val Arg Asn Phe Val Pro Ala His Glu Gln
    130                 135                 140

Ile Ile Asn His Asp Trp Glu Val Ala Ala Ile Ala Lys Asp Ala Tyr
145                 150                 155                 160
```

Asp Ile Glu Gly Lys Thr Ile Ala Thr Ile Gly Ala Gly Arg Ile Gly
165 170 175

Tyr Gly Val Leu Glu Arg Leu Leu Pro Phe Asn Pro Lys Glu Leu Leu
180 185 190

Tyr Tyr Asp Tyr Gln Ala Leu Pro Lys Glu Ala Glu Glu Lys Val Gly
195 200 205

Ala Arg Arg Val Glu Asn Ile Glu Glu Leu Val Ala Gln Ala Asp Ile
210 215 220

Val Thr Val Asn Ala Pro Leu His Ala Gly Thr Lys Gly Leu Ile Asn
225 230 235 240

Lys Glu Leu Leu Ser Lys Phe Lys Lys Gly Ala Trp Leu Val Asn Thr
245 250 255

Ala Arg Gly Ala Ile Cys Val Ala Glu Asp Val Ala Ala Ala Leu Glu
260 265 270

Ser Gly Gln Leu Arg Gly Tyr Gly Gly Asp Val Trp Phe Pro Gln Pro
275 280 285

Ala Pro Lys Asp His Pro Trp Arg Asp Met Arg Asn Lys Tyr Gly Ala
290 295 300

Gly Asn Ala Met Thr Pro His Tyr Ser Gly Thr Thr Leu Asp Ala Gln
305 310 315 320

Thr Arg Tyr Ala Glu Gly Thr Lys Asn Ile Leu Glu Ser Phe Phe Thr
325 330 335

Gly Lys Phe Asp Tyr Arg Pro Gln Asp Ile Ile Leu Leu Asn Gly Glu
340 345 350

Tyr Val Thr Lys Ala Tyr Gly Lys His Asp Lys Lys
355 360

<210> 37
<211> 364
<212> PRT
<213> Künstliche Sequenz

<220>
<223> Beschreibung der künstlichen Sequenz:SM2pC7

<400> 37
Met Lys Ile Val Leu Val Leu Tyr Asp Ala Gly Lys His Ala Ala Asp
1 5 10 15

Glu Glu Lys Leu Tyr Gly Ser Thr Glu Asn Lys Leu Gly Ile Ala Asn
20 25 30

Trp Leu Lys Asp Gln Gly His Glu Leu Ile Thr Thr Ser Asp Lys Glu
35 40 45

Gly Glu Thr Ser Glu Leu Asp Lys His Ile Pro Asp Ala Asp Ile Ile

50 55 60

Ile Thr Thr Pro Phe His Pro Ala Tyr Ile Thr Lys Glu Arg Leu Asp
65 70 75 80

Lys Ala Lys Asn Leu Lys Leu Val Val Val Ala Gly Val Gly Ser Asp
85 90 95

His Ile Asp Leu Asp Tyr Ile Asn Gln Thr Gly Lys Lys Ile Ser Val
100 105 110

Leu Glu Val Thr Gly Ser Asn Val Val Ser Val Ala Glu His Val Val
115 120 125

Met Thr Met Leu Val Leu Val Arg Asn Phe Val Pro Ala His Glu Gln
130 135 140

Ile Ile Asn His Glu Trp Glu Val Ala Ala Ile Ala Lys Asp Ala Tyr
145 150 155 160

Asp Ile Glu Gly Lys Thr Ile Ala Thr Ile Gly Ala Gly Arg Ile Gly
165 170 175

Tyr Ser Val Leu Glu Arg Leu Leu Pro Phe Asn Pro Lys Glu Leu Leu
180 185 190

Tyr Tyr Asp Tyr Gln Ala Leu Pro Lys Glu Ala Glu Glu Lys Val Gly
195 200 205

Ala Arg Arg Val Glu Asn Ile Glu Glu Leu Val Ala Gln Ala Asp Ile
210 215 220

Val Thr Val Asn Ala Pro Leu His Ala Gly Thr Lys Gly Leu Ile Asn
225 230 235 240

Lys Glu Leu Leu Ser Lys Phe Lys Lys Gly Ala Trp Leu Val Asn Thr
245 250 255

Ala Arg Gly Ala Ile Cys Val Ala Glu Asp Val Ala Ala Ala Leu Glu
260 265 270

Ser Gly Gln Leu Arg Gly Tyr Gly Gly Asp Val Trp Phe Pro Gln Pro
275 280 285

Ala Pro Lys Asp His Pro Trp Arg Asp Met Arg Asn Lys Tyr Gly Ala
290 295 300

Gly Asn Ala Met Thr Pro His Tyr Ser Gly Thr Thr Leu Asp Ala Gln
305 310 315 320

Thr Arg Tyr Ala Glu Gly Thr Lys Asn Ile Leu Glu Ser Phe Phe Thr
325 330 335

Gly Lys Phe Asp Tyr Arg Pro Gln Asp Ile Ile Leu Leu Asn Gly Glu
340 345 350

Tyr Val Thr Lys Ala Tyr Gly Lys His Asp Lys Lys
355 360

<210> 38
<211> 364
<212> PRT
<213> Künstliche Sequenz

<220>
<223> Beschreibung der künstlichen Sequenz:SM-4cA10

<400> 38
Met Lys Ile Val Leu Val Leu Tyr Asp Ala Gly Lys His Ala Ala Asp
1 5 10 15

Glu Glu Lys Leu Tyr Gly Ser Thr Glu Asn Lys Leu Gly Ile Ala Asn
20 25 30

Trp Leu Lys Asp Gln Gly His Glu Leu Ile Thr Thr Ser Asp Lys Glu
35 40 45

Gly Glu Thr Ser Glu Leu Asp Lys His Ile Pro Asp Ala Asp Ile Ile
50 55 60

Ile Thr Thr Pro Phe His Pro Ala Tyr Ile Thr Lys Glu Arg Leu Asp
65 70 75 80

Lys Ala Lys Asn Leu Lys Leu Val Val Val Ala Gly Val Gly Ser Asp
85 90 95

His Ile Asp Leu Asp Tyr Ile Asn Gln Thr Gly Lys Lys Ile Ser Val
100 105 110

Leu Glu Val Thr Gly Ser Asn Val Val Ser Val Ala Glu His Val Val
115 120 125

Met Thr Met Leu Val Leu Val Arg Asn Phe Val Pro Ala His Glu Gln
130 135 140

Ile Ile Asn His Asp Trp Asp Val Ala Ala Ile Ala Lys Asp Ala Tyr
145 150 155 160

Asp Ile Glu Gly Lys Thr Ile Ala Thr Ile Gly Ala Gly Arg Ile Gly
165 170 175

Tyr Ser Val Leu Glu Arg Leu Leu Pro Phe Asn Pro Lys Glu Leu Leu
180 185 190

Tyr Tyr Asp Tyr Gln Ala Leu Pro Lys Glu Ala Glu Glu Arg Val Gly
195 200 205

Ala Arg Arg Val Glu Asn Ile Glu Glu Leu Val Ala Gln Ala Asp Ile
210 215 220

Val Thr Val Asn Ala Pro Leu His Ala Gly Thr Lys Gly Leu Ile Asn
225 230 235 240

Lys Glu Leu Leu Ser Lys Phe Lys Lys Gly Ala Trp Leu Val Asn Thr
245 250 255

Ala Arg Gly Ala Ile Cys Val Ala Glu Asp Val Ala Ala Ala Leu Glu

260 265 270

Ser Gly Gln Leu Arg Gly Tyr Gly Gly Asp Val Trp Phe Pro Gln Pro
275 280 285

Ala Pro Lys Asp His Pro Trp Arg Asp Met Arg Asn Lys Tyr Gly Ala
290 295 300

Gly Asn Ala Met Thr Pro His Tyr Ser Gly Asn Thr Leu Asp Ala Gln
305 310 315 320

Thr Arg Tyr Ala Glu Gly Thr Lys Asn Ile Leu Glu Ser Phe Phe Thr
325 330 335

Gly Lys Phe Asp Tyr Arg Pro Gln Asp Ile Ile Leu Leu Asn Gly Glu
340 345 350

Tyr Val Thr Lys Ala Tyr Gly Lys His Asp Lys Lys
355 360

<210> 39
<211> 364
<212> PRT
<213> Künstliche Sequenz

<220>
<223> Beschreibung der künstlichen Sequenz:SM-4fD3

<400> 39
Met Lys Ile Val Leu Val Leu Tyr Asp Ala Gly Lys His Ala Ala Asp
1 5 10 15

Glu Glu Lys Leu Tyr Gly Ser Thr Glu Asn Lys Leu Gly Ile Ala Asn
20 25 30

Trp Leu Lys Asp Gln Gly His Glu Leu Ile Thr Thr Ser Asp Lys Glu
35 40 45

Gly Glu Thr Ser Glu Leu Asp Lys His Ile Pro Asp Ala Asp Ile Ile
50 55 60

Ile Thr Thr Pro Phe His Pro Ala Tyr Ile Thr Lys Glu Arg Leu Asp
65 70 75 80

Lys Ala Lys Asn Leu Lys Leu Val Val Val Ala Gly Val Gly Ser Asp
85 90 95

His Ile Asp Leu Asp Tyr Ile Asn Gln Thr Gly Lys Lys Ile Ser Val
100 105 110

Leu Glu Val Thr Gly Ser Asn Val Val Ser Val Ala Glu His Val Val
115 120 125

Met Thr Met Leu Val Leu Val Arg Asn Phe Val Pro Ala His Glu Gln
130 135 140

Ile Ile Asn His Asp Trp Asp Val Ala Ala Ile Ala Lys Asp Ala Tyr
145 150 155 160

```
Asp Ile Glu Gly Lys Thr Ile Ala Thr Ile Gly Ala Gly Arg Ile Gly
                165                 170                 175

Tyr Ser Val Leu Glu Arg Leu Leu Pro Phe Asn Pro Lys Glu Leu Leu
            180                 185                 190

Tyr Tyr Asp Tyr Gln Ala Leu Pro Lys Glu Ala Glu Glu Lys Val Gly
        195                 200                 205

Ala Arg Arg Val Glu Asn Ile Glu Glu Leu Val Ala Gln Ala Asp Ile
    210                 215                 220

Val Thr Val Asn Ala Pro Leu His Ala Gly Thr Lys Gly Leu Ile Asn
225                 230                 235                 240

Lys Glu Leu Leu Ser Lys Phe Lys Lys Gly Ala Trp Leu Val Asn Thr
                245                 250                 255

Ala Arg Gly Ala Ile Cys Val Ala Glu Asp Val Ala Ala Ala Leu Glu
            260                 265                 270

Ser Gly Gln Leu Arg Gly Tyr Gly Gly Asp Val Trp Phe Pro Gln Pro
        275                 280                 285

Ala Pro Lys Asp His Pro Trp Arg Asp Met Arg Asn Lys Tyr Gly Ala
    290                 295                 300

Gly Asn Ala Met Thr Pro His Tyr Ser Gly Thr Thr Leu Asp Ala Gln
305                 310                 315                 320

Thr Arg Tyr Ala Glu Gly Thr Lys Asn Ile Leu Glu Ser Phe Phe Thr
                325                 330                 335

Gly Lys Phe Asp Tyr Arg Pro Gln Asp Ile Ile Leu Leu Asn Gly Glu
            340                 345                 350

Tyr Val Thr Lys Ala Tyr Gly Lys His Asp Lys Lys
        355                 360


<210> 40
<211> 364
<212> PRT
<213> Künstliche Sequenz

<220>
<223> Beschreibung der künstlichen Sequenz:SM-4sG4

<400> 40
Met Lys Ile Val Leu Val Leu Tyr Asp Ala Gly Lys His Ala Ala Asp
  1               5                  10                  15

Glu Glu Lys Leu Tyr Gly Ser Thr Glu Asn Lys Leu Gly Ile Ala Asn
            20                  25                  30

Trp Leu Lys Asp Gln Gly His Glu Leu Ile Thr Thr Ser Asp Lys Glu
        35                  40                  45
```

```
Gly Glu Thr Ser Glu Leu Asp Lys His Ile Pro Asp Ala Asp Ile Ile
     50                  55                  60

Ile Thr Thr Pro Phe His Pro Ala Tyr Ile Thr Lys Glu Arg Leu Asp
65                  70                  75                  80

Lys Ala Lys Asn Leu Lys Leu Val Val Val Ala Gly Val Gly Ser Asp
                85                  90                  95

His Ile Asp Leu Asp Tyr Ile Asn Gln Thr Gly Lys Lys Ile Ser Val
            100                 105                 110

Leu Glu Val Thr Gly Ser Asn Val Val Ser Val Ala Glu His Val Val
        115                 120                 125

Met Thr Met Leu Val Leu Val Arg Asn Phe Val Pro Ala His Glu Gln
    130                 135                 140

Ile Ile Asn His Asp Trp Asp Val Ala Ala Ile Ala Lys Asp Ala Tyr
145                 150                 155                 160

Asp Ile Glu Gly Lys Thr Ile Ala Thr Ile Gly Ala Gly Arg Ile Gly
                165                 170                 175

Tyr Ser Val Leu Glu Arg Leu Leu Pro Phe Asn Pro Lys Glu Leu Leu
            180                 185                 190

Tyr Tyr Asp Tyr Gln Ala Leu Pro Lys Glu Ala Glu Glu Lys Val Gly
        195                 200                 205

Ala Arg Arg Val Glu Asn Ile Glu Glu Leu Val Ala Gln Ala Asp Ile
    210                 215                 220

Val Thr Val Asn Ala Pro Leu His Ala Gly Thr Lys Gly Leu Ile Asn
225                 230                 235                 240

Lys Glu Leu Leu Ser Lys Phe Lys Lys Gly Ala Trp Leu Val Asn Thr
                245                 250                 255

Ala Arg Gly Ala Ile Cys Val Ala Glu Asp Val Ala Ala Ala Leu Glu
            260                 265                 270

Ser Gly Gln Leu Arg Gly Tyr Gly Gly Asp Val Trp Phe Pro Gln Pro
        275                 280                 285

Ala Pro Lys Asp His Pro Trp Arg Asp Met Arg Asn Lys Tyr Gly Ala
    290                 295                 300

Gly Asn Ala Met Thr Pro His Tyr Ser Gly Thr Thr Leu Asp Ala Gln
305                 310                 315                 320

Thr Arg Tyr Ala Glu Gly Thr Lys Asn Ile Leu Glu Ser Phe Phe Thr
                325                 330                 335

Gly Lys Phe Asp Tyr Arg Pro Gln Asp Ile Ile Leu Leu Asn Gly Glu
            340                 345                 350

Tyr Val Thr Glu Ala Tyr Gly Lys His Asp Lys Lys
        355                 360
```

<210> 41
<211> 364
<212> PRT
<213> Künstliche Sequenz

<220>
<223> Beschreibung der künstlichen Sequenz:SM-4sG6

<400> 41
Met Lys Ile Val Leu Val Leu Tyr Asp Ala Gly Lys His Ala Ala Asp
1 5 10 15

Glu Glu Lys Leu Tyr Gly Ser Thr Glu Asn Lys Leu Gly Ile Ala Asn
20 25 30

Trp Leu Lys Asp Gln Gly His Glu Leu Ile Thr Thr Ser Asp Lys Glu
35 40 45

Gly Glu Thr Ser Glu Leu Asp Lys His Ile Pro Asp Ala Asp Ile Ile
50 55 60

Ile Thr Thr Pro Phe His Pro Ala Tyr Ile Thr Lys Glu Arg Leu Asp
65 70 75 80

Lys Ala Lys Asn Leu Lys Leu Val Val Val Ala Gly Val Gly Ser Asp
85 90 95

His Ile Asp Leu Asp Tyr Ile Asn Gln Thr Gly Lys Lys Ile Ser Val
100 105 110

Leu Glu Val Thr Gly Ser Asn Val Val Ser Val Ala Glu His Val Val
115 120 125

Met Thr Met Leu Val Leu Val Arg Asn Phe Val Pro Ala His Glu Gln
130 135 140

Ile Ile Asn His Asp Trp Asp Val Ala Ala Ile Ala Lys Asp Ala Tyr
145 150 155 160

Asp Ile Glu Gly Lys Thr Ile Ala Thr Ile Gly Ala Gly Arg Ile Gly
165 170 175

Tyr Ser Val Leu Glu Arg Leu Leu Pro Phe Asn Pro Lys Glu Leu Leu
180 185 190

Tyr Tyr Asp Tyr Gln Ala Leu Pro Lys Glu Ala Glu Glu Arg Val Gly
195 200 205

Ala Arg Arg Val Glu Asn Ile Glu Glu Leu Val Ala Gln Ala Asp Ile
210 215 220

Val Thr Val Asn Ala Pro Leu His Ala Gly Thr Lys Gly Leu Ile Asn
225 230 235 240

Lys Glu Leu Leu Ser Lys Phe Lys Lys Gly Ala Trp Leu Val Asn Thr
245 250 255

```
Ala Arg Gly Ala Ile Cys Val Ala Glu Asp Val Ala Ala Ala Leu Glu
            260                 265                 270

Ser Gly Gln Leu Arg Gly Tyr Gly Gly Asp Val Trp Phe Pro Gln Pro
        275                 280                 285

Ala Pro Lys Asp His Pro Trp Arg Asp Met Arg Asn Lys Tyr Gly Ala
    290                 295                 300

Gly Asn Ala Met Thr Pro His Tyr Ser Gly Thr Thr Leu Asp Ala Gln
305                 310                 315                     320

Thr Arg Tyr Ala Glu Gly Thr Lys Asn Ile Leu Glu Ser Phe Phe Thr
                325                 330                 335

Gly Lys Phe Asp Tyr Arg Pro Gln Asp Ile Ile Leu Leu Asn Gly Glu
            340                 345                 350

Tyr Val Thr Glu Ala Tyr Gly Lys His Asp Lys Lys
        355                 360


<210> 42
<211> 364
<212> PRT
<213> Künstliche Sequenz

<220>
<223> Beschreibung der künstlichen Sequenz:SM-F285S

<400> 42
Met Lys Ile Val Leu Val Leu Tyr Asp Ala Gly Lys His Ala Ala Asp
  1               5                  10                  15

Glu Glu Lys Leu Tyr Gly Ser Thr Glu Asn Lys Leu Gly Ile Ala Asn
            20                  25                  30

Trp Leu Lys Asp Gln Gly His Glu Leu Ile Thr Thr Ser Asp Lys Glu
        35                  40                  45

Gly Glu Thr Ser Glu Leu Asp Lys His Ile Pro Asp Ala Asp Ile Ile
    50                  55                  60

Ile Thr Thr Pro Phe His Pro Ala Tyr Ile Thr Lys Glu Arg Leu Asp
 65                  70                  75                      80

Lys Ala Lys Asn Leu Lys Leu Val Val Val Ala Gly Val Gly Ser Asp
                85                  90                  95

His Ile Asp Leu Asp Tyr Ile Asn Gln Thr Gly Lys Lys Ile Ser Val
            100                 105                 110

Leu Glu Val Thr Gly Ser Asn Val Val Ser Val Ala Glu His Val Val
        115                 120                 125

Met Thr Met Leu Val Leu Val Arg Asn Phe Val Pro Ala His Glu Gln
    130                 135                 140

Ile Ile Asn His Asp Trp Glu Val Ala Ala Ile Ala Lys Asp Ala Tyr
```

```
145                 150                 155                 160

Asp Ile Glu Gly Lys Thr Ile Ala Thr Ile Gly Ala Gly Arg Ile Gly
                165                 170                 175

Tyr Arg Val Leu Glu Arg Leu Leu Pro Phe Asn Pro Lys Glu Leu Leu
            180                 185                 190

Tyr Tyr Asp Tyr Gln Ala Leu Pro Lys Glu Ala Glu Glu Lys Val Gly
        195                 200                 205

Ala Arg Arg Val Glu Asn Ile Glu Glu Leu Val Ala Gln Ala Asp Ile
    210                 215                 220

Val Thr Val Asn Ala Pro Leu His Ala Gly Thr Lys Gly Leu Ile Asn
225                 230                 235                 240

Lys Glu Leu Leu Ser Lys Phe Lys Lys Gly Ala Trp Leu Val Asn Thr
                245                 250                 255

Ala Arg Gly Ala Ile Cys Val Ala Glu Asp Val Ala Ala Ala Leu Glu
            260                 265                 270

Ser Gly Gln Leu Arg Gly Tyr Gly Gly Asp Val Trp Ser Pro Gln Pro
        275                 280                 285

Ala Pro Lys Asp His Pro Trp Arg Asp Met Arg Asn Lys Tyr Gly Ala
    290                 295                 300

Gly Asn Ala Met Thr Pro His Tyr Ser Gly Thr Thr Leu Asp Ala Gln
305                 310                 315                 320

Thr Arg Tyr Ala Glu Gly Thr Lys Asn Ile Leu Glu Ser Phe Phe Thr
                325                 330                 335

Gly Lys Phe Asp Tyr Arg Pro Gln Asp Ile Ile Leu Leu Asn Gly Glu
            340                 345                 350

Tyr Val Thr Lys Ala Tyr Gly Lys His Asp Lys Lys
        355                 360


<210> 43
<211> 364
<212> PRT
<213> Künstliche Sequenz

<220>
<223> Beschreibung der künstlichen Sequenz:
      Kombinationsmutante SM-4fD3-F285S

<400> 43
Met Lys Ile Val Leu Val Leu Tyr Asp Ala Gly Lys His Ala Ala Asp
  1               5                  10                  15

Glu Glu Lys Leu Tyr Gly Ser Thr Glu Asn Lys Leu Gly Ile Ala Asn
            20                  25                  30

Trp Leu Lys Asp Gln Gly His Glu Leu Ile Thr Thr Ser Asp Lys Glu
```

35 40 45

Gly Glu Thr Ser Glu Leu Asp Lys His Ile Pro Asp Ala Asp Ile Ile
50 55 60

Ile Thr Thr Pro Phe His Pro Ala Tyr Ile Thr Lys Glu Arg Leu Asp
65 70 75 80

Lys Ala Lys Asn Leu Lys Leu Val Val Val Ala Gly Val Gly Ser Asp
85 90 95

His Ile Asp Leu Asp Tyr Ile Asn Gln Thr Gly Lys Lys Ile Ser Val
100 105 110

Leu Glu Val Thr Gly Ser Asn Val Val Ser Val Ala Glu His Val Val
115 120 125

Met Thr Met Leu Val Leu Val Arg Asn Phe Val Pro Ala His Glu Gln
130 135 140

Ile Ile Asn His Asp Trp Asp Val Ala Ala Ile Ala Lys Asp Ala Tyr
145 150 155 160

Asp Ile Glu Gly Lys Thr Ile Ala Thr Ile Gly Ala Gly Arg Ile Gly
165 170 175

Tyr Ser Val Leu Glu Arg Leu Leu Pro Phe Asn Pro Lys Glu Leu Leu
180 185 190

Tyr Tyr Asp Tyr Gln Ala Leu Pro Lys Glu Ala Glu Glu Lys Val Gly
195 200 205

Ala Arg Arg Val Glu Asn Ile Glu Glu Leu Val Ala Gln Ala Asp Ile
210 215 220

Val Thr Val Asn Ala Pro Leu His Ala Gly Thr Lys Gly Leu Ile Asn
225 230 235 240

Lys Glu Leu Leu Ser Lys Phe Lys Lys Gly Ala Trp Leu Val Asn Thr
245 250 255

Ala Arg Gly Ala Ile Cys Val Ala Glu Asp Val Ala Ala Ala Leu Glu
260 265 270

Ser Gly Gln Leu Arg Gly Tyr Gly Gly Asp Val Trp Ser Pro Gln Pro
275 280 285

Ala Pro Lys Asp His Pro Trp Arg Asp Met Arg Asn Lys Tyr Gly Ala
290 295 300

Gly Asn Ala Met Thr Pro His Tyr Ser Gly Thr Thr Leu Asp Ala Gln
305 310 315 320

Thr Arg Tyr Ala Glu Gly Thr Lys Asn Ile Leu Glu Ser Phe Phe Thr
325 330 335

Gly Lys Phe Asp Tyr Arg Pro Gln Asp Ile Ile Leu Leu Asn Gly Glu
340 345 350

```
Tyr Val Thr Lys Ala Tyr Gly Lys His Asp Lys Lys
        355                 360
```

## Beschreibung

Die vorliegende Erfindung ist auf neue Mutanten einer rec-FDH aus *Candida boidinii* (ATCC 32195) gerichtet. Weiterhin beschreibt die Erfindung die für diese Mutanten codierenden Nukleinsäuren sowie Vehikel, welche diese Nukleinsäuren enthalten. Darüberhinaus wird auch ein Verfahren zur Herstellung von weiterhin verbesserten FDHs erwähnt sowie ein Verfahren zum vorteilhaften Screening von stabileren und aktiveren Dehydrogenasen.

Zur Herstellung von L-Aminosäuren werden u. a. erfolgreich Biokatalysatoren eingesetzt. Ein Ansatz geht dabei von der Umwandlung prochiraler α-Ketosäuren durch reduktive. Aminierung aus. Die dabei benutzten Aminosäuredehydrogenasen benötigen zur Umsetzung der α-Ketosäuren stöchiometrische Mengen an NADH bzw. NADPH als Coenzym. Diese Coenzyme sind sehr teuer und machen dadurch das o. g. Verfahren für den industriellen Maßstab wirtschaftlich uninteressant.

Eine Möglichkeit, hohe Kosten durch das Coenzym zu vermeiden, besteht darin, das Coenzym *in situ* zu regenerieren. Im technischen Maßstab wird derzeit u. a. die NAD-abhängige Formiatdehydrogenase aus der Hefe *Candida boidinii* im Enzymreaktor zur Coenzymregeneration eingesetzt.

Schema 1:

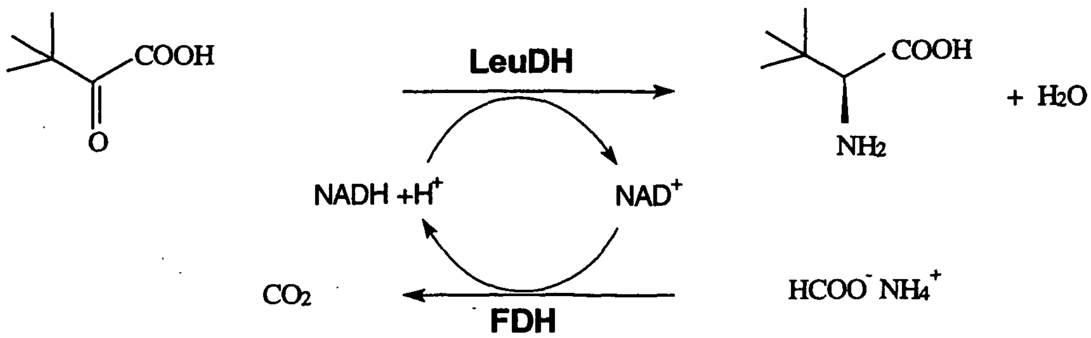


Schema 1 zeigt die *in situ* Regeneration von NADH mit der NAD-abhängigen Formiatdehydrogenase bei der reduktiven Aminierung von Trimethylpyruvat zu L-tert-Leucin (Bommarius et al., Tetrahedron Asymmetry 1995, 6, 2851-2888).

Ein Nachteil, den der Einsatz der FDH aus Candida boidinii im Produktionsprozeß mit sich bringt, ist die Notwendigkeit, FDH während des Prozesses nachdosieren zu müssen, da sie aufgrund fehlender Stabilität inaktiviert.

Diese Inaktivierung kann durch verschiedene Faktoren beeinflußt werden:

- pH-Wert
- Temperatur
- mechanische Belastung
- Ionenstärke und Ionenart der Substratlösung
- Spuren von Schwermetallen
- Oxidation von Sulfhydrylgruppen durch Luftsauerstoff
- Vernetzung durch Thiol-Disulfidaustausch

Tishkov et al. zeigten, daß mittels gerichteter Mutation der rekombinanten FDH aus *Pseudomonas sp.* 101 deren Stabilität gegenüber Quecksilbersalzen erhöht wird, wohingegen die thermische Stabilität jedoch durch die Mutagenese erniedrigt wurde (Biochem, Biophys. Res. Commun. 1993, 192, 976-981).

Sakai et al. klärten die Gensequenz der FDH aus der methylotrophen Hefe *Candida boidinii* auf (J. Bacteriol. 1997, 179, 4480-4485), die abgeleitete Proteinsequenz stimmt zu 100 % mit der Aminosäuresequenz der rekombinanten FDH aus *Candida boidinii* überein.

In der DE19753350 ist neben anderen Mutanten der Formiatdehydrogenase aus Candida boidinii auch das an Stelle 23 mit Serin als Aminosäure versehene (C23S) Protein und das zusätzlich an Stelle 262 mit Alanin versehene

(C23S/C262A) beschrieben. Diese zeigen eine höhere Stabilität im Hinblick auf die Aggregations- und Oxidationsempfindlichkeit als das native bzw. rekombinant (rec-) hergestellte Enzym, besitzen jedoch keine gesteigerte katalytische Aktivität und Thermostabilität.

Alle bisher beschriebenen NAD-abhängigen Formiatdehydrogenasen (EC 1.2.1.2) zeichnen sich durch relativ geringe spezifische Aktivitäten zwischen 5 und 7 U/mg Protein bei 30°C aus (Popov, V.O., Lamzin, V.S. (1994) NAD+-dependent formate dehydrogenase. Biochem. J. 130, 625-643). Im Vergleich dazu hat die zur Herstellung des L-tertiär Leucins (Schema 1) verwendete Leucindehydrogenase (LeuDH) eine spezifische Aktivität von ~ 200 U/mg. Um somit eine stöchiometrische Regeneration von NADH zu gewährleisten, muß eine vielfache Menge an FDH-Protein im Vergleich zur LeuDH bereitgestellt und eingesetzt werden.

Angesicht des hierin angegebenen und diskutierten Standes der Technik war es mithin Aufgabe der vorliegenden Erfindung, sowohl die katalytische Aktivität als auch die Stabilität der oxidations- und aggregationsunempfindlicheren rec-FDH aus Candida boidinii mit der C23S-Mutation bzw. der C23S/C262A-Doppelmutation weiterhin zu erhöhen, um für den Einsatz in einem technischen Prozeß geringere Enzymmengen herstellen und einsetzen zu müssen und ein kostspieliges Nachdosieren der FDH während des Prozesses zu vermeiden, was Produktionskosten sparen hülfe.

Diese und weitere nicht näher spezifizierte sich jedoch aus dem Stand der Technik in naheliegender Weise ergebende Aufgaben werden durch die Mutanten nach Anspruch 1 gelöst. Anspruch 2 bezieht sich auf weitere erfindungsgemäß mutierte FDHs. Anspruch 3 ist auf die diese Mutanten codierenden Nukleinsäuren gerichtet, während Anspruch 4 Vehikel schützt, die diese Nukleinsäuren beherbergen. Anspruch 5 nennt spezielle Primer. Ansprüche 6 bis 9 beziehen sich auf ein Verfahren zur Herstellung weiterhin verbesserter Muteine basierend auf den hier vorgestellten Mutanten, die durch ein solches Verfahren gewonnenen Enzyme, diese codierende Nukleinsäuren und deren Verwendung. Weiterhin wird in Ansprüchen 10 bis 12 ein vorteilhafter Ganzzellkatalysator beansprucht. Anspruch 13 richtet sich auf ein Screeningverfahren für aktivere und stabilere Dehydrogenasen.

Durch die Bereitstellung von gegenüber der Wildtyp-rec-FDH und dem nativen Wildtypenzym aus *Candida boidinii* stabilere und/oder katalytisch aktivere Mutanten, wobei diese Mutanten den Aminosäureaustausch C23S (DE19753350; Seq. 1) oder C23S/C262A (DE19753350; Seq. 3) und weiterhin einen oder mehrere der folgenden Aminosäureaustausche aufweisen: E18D, K35R, D149E, E151D, R178S, R178G, K206R, F285Y, F285S, T315N, K356E, gelangt man zu verbesserten Biokatalysatoren, welche vorteilhaft z.B. in einem wie eingangs erwähnten technischen Prozeß einzusetzen sind, beziehungsweise in einem Verfahren zur Herstellung von chiralen Verbindungen, insbesondere Aminosäuren (natürliche und unnatürliche) in optisch angereicherter (enantiomerenangereicherter) Form verwendet werden können. Optional können die Mutanten zusätzlich auch die Mutation C262A aufweisen.

Es liegt nahe, diese vorteilhaften erfindungsgemäßen Aminosäureaustausche auch in anderen FDHs, welche nicht aus *Candida boidinii* stammen, aber eine entsprechend homologe Sequenz aufweisen ebenfalls einzufügen. Daher betrifft die Erfindung ebenfalls Aminosäuresequenzen mit FDH-Aktivität, welche gegenüber der Wildtyp-rec-FDH und dem nativen Wildtypenzym aus *Candida boidinii* stabiler und/oder aktiver sind, aufweisend einen oder mehrere der folgenden Aminosäureaustausche: 18D, 35R, 149E, 151D, 178S, 178G, 206R, 285Y, 285S, 315N, 356E, wobei die Austausche an den entsprechenden äquivalenten Positionen in der Sequenz erfolgen.

Die Positionsnummern von Aminosäuren werden durch fortlaufende Nummerierung der Aminosäuren beginnend beim Startcodon der Sequenz vergeben. Daher können Aminosäuren, welche in gleicher Weise die Enzymfunktion beeinflussen, aufgrund von Deletionen oder Insertionen durchaus unterschiedliche Positionsnummern in gleich gearteten Enzymen besitzen. Eine äquivalente Position in gleichartigen Enzymen trägt in gleichgearteter Weise zur Änderung der Aktivität und Stabilität bei, wie in der Ausgangsmutante C23S. Sofern eine hohe Sequenzhomologie zwischen den gleichartigen Enzymen besteht, z.B. > 60%, kann zum Auffinden der entsprechenden äquivalenten Positionen zwischen der Mutante aus *Candida boidinii* und der zu mutierenden Aminosäuresequenz ein sogenanntes Alignment, z. B. mit dem Programm BLAST® (J. Mol. Biol. 1990, 215, 403-410), durchgeführt werden. Dabei werden konservierte Bereiche in den zu vergleichenden Sequenzen direkt untereinandergestellt. Eine entsprechende äquivalente Position ergibt sich aus den den oben genannten Positionen entsprechenden Positionen auf dem Vergleichsstrang unter Berücksichtigung der Tatsache, daß der dortige Austausch in gleichgearteter Weise zur Änderung der Aktivität und Stabilität beiträgt, wie in der C23S-Mutante. Eine weitere Möglichkeit, äquivalente Positionen ausfindig zu machen, bietet der Vergleich von Röntgenstrukturuntersuchungen (Cur. Opin. Struc. Biol. 1995, 5, 377-382), bei dem durch Übereinanderlegen von 3-D-Strukturen von Enzymen derartige Positionen ausfindig gemacht werden können. Behilflich können in diesem Zusammenhang auch empirische und semiempirische Strukturanalyseprogramme sein. Die Austausche in den Aminosäuresequenzen mit FDH-Aktivität können durch dem Fachmann bekannte und weiter unten angegebene Mutagenesemethoden und Einsatz rekombinanter Techniken (Lit. s.u.) erfolgen.

In einer nächsten Ausgestaltung beschäftigt sich die Erfindung mit Nukleinsäuren aufweisend eine Sequenz codierend für eine erfindungsgemäße Mutante der FDH aus *C. boidinii* und den eben genannten Aminosäuresequenzen mit FDH-Aktivität. Mitumfaßt sind auch erfindungsgemäße Nukleinsäuren, welche über die eigentliche codierende Sequenz hinaus z.B. die für Restriktionsenzyme wichtigen Sequenzen, Tag-Sequenzen (His-, Mal-) oder Transkriptionsterminatoren (rrnB) aufweisen.

Durch die Angabe der Nukleinsäuren gelangt man in vorteilhafter Art und Weise zu Substanzen, welche es erlauben, die für einen wie eingangs erwähnten enzymatischtechnischen Prozeß zur Erzeugung von z.B. Aminosäuren notwendigen Enzyme in ausreichender Menge sicherzustellen. Über bekannte rekombinante Techniken (s. unten) ist es mit den erfindungsgemäßen Nukleinsäuren möglich, die Enzyme aus schnell wachsenden Wirtsorganismen in hohen Ausbeuten zu gewinnen. Außerdem sind die erfindungsgemäßen Gensequenzen zur Erzeugung von ggf. weiterhin verbesserten Mutanten zu verwenden. Durch besagte rekombinante Techniken, die dem Fachmann hinlänglich bekannt sind (s.u.), gelangt man zu Organismen, welche in der Lage sind, das betrachtete Enzym in für einen technischen Prozeß ausreichender Menge zur Verfügung zu stellen. Die Herstellung der erfindungsgemäßen rec-Enzyme erfolgt nach dem Fachmann bekannten gentechnologischen Verfahren (Sambrook et al. 1989, Molecular cloning: A Laboratory Manual, 2nd Edition, Cold Spring Harbor Laboratory Press, Balbas P & Bolivar F. 1990; Design and construction of expression plasmid vectors in E.coli, Methods Enzymology 185, 14-37; Vectors: A Survey of Molecular Cloning Vectors and Their Uses. R.L. Rodriguez & D.T. Denhardt, Eds: 205-225). Bezüglich der allgemeinen Vorgehensweise (PCR, Klonierung, Expression etc.) sei auch auf folgende Literatur und das dort zitierte verwiesen: Sambrook J, Fritsch EF, Maniatis T (1989). Molecular Cloning. Cold Spring Harbor Laboratory Press; Vectors: A Survey of Molecular Cloning Vectors and Their Uses. R.L. Rodriguez & D.T. Denhardt, II. Eine weitere Erfindung bezieht sich auf Plasmide oder Vektoren aufweisend eine oder mehrere der erfindungsgemäßen Nukleinsäuren.

Als Plasmide oder Vektoren kommen im Prinzip alle dem Fachmann für diesen Zweck zur Verfügung stehenden Ausführungsformen in Frage. Derartige Plasmide und Vektoren können Studier et al., Methods Enzymol. 1990, 185, 61-69 oder den Broschüren der Firmen Roche Biochemicals, Invitrogen, Novagen, Promega, New England Biolabs, Clontech oder Gibco BRL entnommen werden. Weiter bevorzugte Plasmide und Vektoren können gefunden werden in: DNA cloning: a practical approach. Volume I-III, edited by D. M. Glover, IRL Press Ltd., Oxford, Washington DC, 1985, 1987; Denhardt, D. T. and Colasanti, J.: A survey of vectors for regulating expression of cloned DNA in E. coli. In: Rodriguez, R.L. and Denhardt, D. T (eds), Vectors, Butterworth, Stoneham, MA, 1987, pp179-204; Gene expression technology. In: Goeddel, D. V. (eds), Methods in Enzymology, Volume 185, Academic Press, Inc., San Diego, 1990; Sambrook, J., Fritsch, E.F. and Maniatis, T. 1989. Molecular cloning: a laboratory manual, 2$^{nd}$ ed. Cold Spring Harbor Laboratory Press, Cold Spring Harbor, N.Y.

Plasmide, mit denen das die erfindungsgemäße Nukleinsäure aufweisende Genkonstrukt in ganz bevorzugter Weise in den Wirtsorganismus kloniert werden kann, sind: pKK-177-3H (Roche Biochemicals), pBTac (Roche Biochemicals), pKK-233-3 (Amersham Pharmacia Biotech), pLex (Invitrogen) oder die Vektoren der pET-Serie (Novagen).

Äußerst bevorzugt sind die Plasmide pBTac-FDH (Fig. 2) sowie pUC-FDH (Fig. 3).

Gleichfalls ist die Erfindung auf Mikroorganismen aufweisend die erfindungsgemäßen Nukleinsäuren gerichtet. Der Mikroorganismus, in den die Nukleinsäuren kloniert werden, dient zur Vermehrung und Gewinnung einer ausreichenden Menge des rekombinanten Enzyms. Die Verfahren hierfür sind dem Fachmann wohlbekannt (Sambrook et al. 1989, Molecular cloning: A Laboratory Manual, 2nd Edition, Cold Spring Harbor Laboratory Press, Balbas P & Bolivar F. 1990, Design and construction of expression plasmid vectors in E.coli, Methods Enzymology 185, 14-37; sowie Lit. s.o. bezüglich rekombinante Techniken). Als Mikroorganismen können im Prinzip alle dem Fachmann für diesen Zweck in Frage kommenden Organismen wie z.B. Prokaryonten oder Eukaryonten, wie Pseudomonas, Streptomyces, Arthrobacter, Bacillus, Staphylococcus, Escherichia, Candida, Hansenula, Pichia, Baculoviren herangezogen werden. Vorzugsweise sind E. coli-Stämme für diesen Zweck zu benutzen. Ganz besonders bevorzugt sind: E. coli NM 522, XL1 Blue, JM101, JM109, JM105, RR1, DH5$\alpha$, TOP 10$^-$ oder HB101. Plasmide, mit denen das die erfindungsgemäße Nukleinsäure aufweisende Genkonstrukt vorzugsweise in den Wirtsorganismus kloniert wird, sind weiter oben angegeben.

Ein folgender Aspekt der Erfindung richtet sich auf Primer zur Herstellung der erfindungsgemäßen Gensequenzen mittels aller Arten von PCR. Mitumfaßt sind die Sense- und

Antisense-Primer codierend für die entsprechenden Aminosäuresequenzen.

Geeignete Primer können prinzipiell nach dem Fachmann bekannten Verfahren gewonnen werden. Das Auffinden der erfindungsgemäßen Primer erfolgt durch Vergleich mit bekannten DNA-Sequenzen oder durch Übersetzung der ins Auge gefaßten Aminosäuresequenzen in das Codon des betrachteten Organismus (z.B. für Streptomyces: Wright et al., Gene 1992, 113, 55-65). Gemeinsamkeiten in der Aminosäuresequenz von Proteinen von sogenannten Superfamilien sind hierfür ebenfalls von Nutzen (Firestine et al., Chemistry & Biology 1996, 3, 779-783). Weitere Informationen diesbezüglich können gefunden werden in Oligonucleotide synthesis: a practical approach, edited by M.J. Gait, IRL Press Ltd, Oxford Washington DC, 1984; PCR Protocols: A guide to methods and applications, edited by M.A. Innis, D.H. Gelfound, J.J. Sninsky and T.J. White. Academic Press, Inc., San Diego, 1990. Bevorzugt sind auch solche Primer, welche gleichzeitig die für Restriktionsenzyme wichtigen Sequenzen in die zu synthetisierende Nukleinsäuresequenz einfügen können.

Ganz besonders bevorzugt sind folgende Primer zur Erzeugung der Mutanten zu verwenden: Tabelle 1

| Primername | Seq. | Seq. Nr. |
|---|---|---|
| PBTACF1 | 5´ TGC CTG GCA GTT CCC TAC TC 3´ | 25 |
| PBTACF2 | 5´ CGT TTC ACT TCT GAG TTC GG 3´ | 26 |
| PBTACR1 | 5´ GGT ATG GCT GTG CAG GTC GT 3´ | 27 |
| PBTACR2 | 5´ CGA CAT CAT AAC GGT TCT GG 3´ | 28 |
| PBTACR3 | 5´ TCA TCG GCT CGT ATA ATG TG 3´ | 29 |
| F285S-F1 | 5'- GGT GAT GTT TGG TCC CCA CAA CCA GCT CCA AAG -3' | 30 |
| F285S-R1 | 5'- GGA GCT GGT TGT GGG GAC CAA ACA TCA CCG TA -3' | 31 |

In einer nächsten Ausgestaltung beschäftigt sich die Erfindung mit einem Verfahren zur Herstellung von weiter verbesserten rec-FDHs ausgehend von Nukleinsäuren codierend für eine der erfindungsgemäßen rec-FDH-Mutanten, wobei man

a) die Nukleinsäuren einer Mutagenese unterwirft,
b) die aus a) erhältlichen Nukleinsäuren in einen geeigneten Vektor kloniert und diesen in ein geeignetes Expressionsystem transferiert und
c) die gebildeten verbesserten Proteine detektiert und isoliert. Dieser Prozeß kann ein oder beliebig viele Male hintereinander ausgeführt werden.

Die Vorgehensweise zur Verbesserung der erfindungsgemäßen Enzyme durch Mutagenese-Methoden ist dem Fachmann hinlänglich bekannt. Als Mutagenese-Methoden kommen alle dem Fachmann für diesen Zweck zur Verfügung stehenden Methoden in Frage. Insbesondere sind dies die Sättigungsmutagenese (A.R,. Oliphant, A.L. Nussbaum, K. Struhl (1986), Cloning of random sequence oligonucleotides, Gene, 44, 177-183), die Zufalls-Mutagenese (R.C. Caldwell, G.F. Joyce (1992), Randomization of genes by PCR mutagenesis, PCR Methods Appl., 2, 28-33), Rekombinationsmethoden wie Shuffling (W.P. Stemmer (1994) DNA shuffling by random fragmentation and reassembly: In vitro recombination for molecular evolution, Proc. Natl. Acad. Sci. USA, 91, 10747-10751), L-shuffling (EP1104457), StEP (H. Zhao, L. Giver, Z. Shao, J. Affholter, F. Arnold (1998) Molecular evolution by staggered extension process (StEP) in vitro recombination, Nat. Biotechnol. 16, 258-261), sowie Site-Directed-Mutagenesis (S.N. Ho, H.D. Hunt, R.M. Horton, J.K. Pullen, L.R. Pease (1989) Site-directed mutagenesis by overlap extension using the polymerase chain reaction, Gene 77, 248-254). Die erhaltenen neuen Nukleinsäuresequenzen werden nach den weiter oben dargestellten Methoden in einen Wirtsorganismus (Lit. s.o.) kloniert und die exprimierten Enzyme mit geeigneten Screening-Methoden (speziell für FDH-Analytik s.u.) detektiert.

Einen ebenfalls erfindungsgemäßen nächsten Aspekt bilden die durch ein wie oben dargestelltes Verfahren erhältlichen verbesserten rec-FDHs sowie die diese rec-FDHs codierenden Nukleinsäuren.

Weiterhin beschäftigt sich die Erfindung ebenfalls mit der Verwendung der erfindungsgemäßen ggf. durch Mutation verbesserten rec-FDHs zur Herstellung von chiralen enantiomerenangereicherten organischen Verbindungen, wie z.B. Alkoholen oder Aminosäuren.

Darüber hinaus eigenen sich die erfindungsgemäßen und weiter verbesserten Nukleinsäuren, die für die betrachteten rec-FDHs codieren, bevorzugt zur Herstellung von Ganzzellkatalysatoren (DE10037115.9 sowie dort zitierte Lit.).

Ebenfalls Gegenstand der Erfindung ist somit auch ein Ganzzellkatalysator aufweisend ein kloniertes Gen für eine Dehydrogenase und ein kloniertes Gen für eine rec-FDH, vorzugsweise aus *Candida boidinii,* besonders bevorzugt eine erfindungsgemäße rec-FDH.
Die Herstellung eines derartigen Organismus ist dem Fachmann geläufig (PCT/EP00/08473; PCT/US00/08159; Lit. s.u.).
Der Vorteil eines solchen Organismus ist die gleichzeitige Expression beider Enzymsysteme, womit nur noch ein rec-Organismus für die Reaktion angezogen werden muß. Um die Expression der Enzyme im Hinblick auf ihre Umsetzungsgeschwindigkeiten abzustimmen, können die entsprechend codierenden Nukleinsäurefragmente auf unterschiedlichen Plasmiden mit unterschiedlichen Kopiezahlen untergebracht werden und/oder unterschiedlich starke Promotoren für eine unterschiedlich starke Expression der Gene verwendet werden. Bei derart abgestimmten Enzymsystemen tritt vorteilhafterweise eine Akkumulation einer ggf. inhibierend wirkenden Zwischenverbindung nicht auf und die betrachtete Reaktion kann in einer optimalen Gesamtgeschwindigkeit ablaufen. Dies ist dem Fachmann jedoch hinlänglich bekannt (PCT/EP00/08473; Gellissen et al,. Appl. Microbiol. Biotechnol. 1996, 46, 46-54).

Einen weiteren jedoch nicht minder vorteilhaften Aspekt der vorliegenden Erfindung bildet ein Verfahren zur Identifizierung aktiverer Mutanten einer NAD- oder NADP-abhängigen Dehydrogenase aufweisend ein quantitatives Screeningverfahren zur Bestimmung der Aktivität, welches im einzelnen folgende Schritte aufweist:

a) die Zellaufschlußlösung der zu vergleichenden Mutanten wird in gleichen Aliquots mit jeweils gleichen Mengen Affinitätschromatographiematerial (feste Phase) zusammengebracht,
b) Trennen des Affinitätschromatographiematerials von den nicht haftenden Bestandteilen,
c) Eluieren der an dem Affinitätschromatographiematerial haftenden Muteine,
d) Bestimmung der Volumenaktivität und Proteinkonzentration und daraus der spezifischen Aktivität.

Das erfindungsgemäße Verfahren erlaubt die Bestimmung der auf die Enzymmenge bezogene spezifische Aktivität (Volumenaktivität/Proteinkonzentration) in besonders einfacher Art und Weise.
Bevorzugt wird die Bestimmung direkt auf sogenannten Mikrotiterplatten durchgeführt. Im einzelnen geht man bei dem Screening so vor, daß man zuerst ein qualitatives Nachweisverfahren direkt auf der Agarplatte durchführt. Hierbei werden mittels einer Aktivitätsfärbung positive Muteine in Gegenwart von Formiat, Phenazinethosulfat, Nitrotetrazoliumblauchlorid und NAD direkt auf der Agarplatte (modifiziert nach O. Gabriel, J. Mol. Biol. 1971, 22, 578-604; A.S. Hawrany et al., J. Mol. Biol. 1996, 264, 97-110), identifiziert. Aktive Klone werden von den Agarplatten gepickt und in 96-well Mikrotiterplatten kultiviert. Die anschließende Expression der FDH-Muteine wird durch Zugabe des Induktors Isopropylthiogalaktosid (IPTG) induziert.
Zur Freisetzung der cytoplasmatischen FDH-Aktivitäten wird die Zellsuspension nach durchschnittlich 16-stündiger Expression mit Triton-X100 / EDTA behandelt, um eine Permeabilisierung der Zellen zu bewirken. Die Isolierung und Reinigung der FDH-Muteine bis zur Homogenität wird mittels einer Ein-Schritt-Affinitätschromatographie (K.H. Kroner et al., J. Chem. Tech. Biotechnol. 1982, 32, 130-137; N.E. Labrou et al., Arch. Biochem. Biophys. 1995, 32, 169-178) in Mikrotiterplatten durchgeführt. Dabei wird die Zellaufschlußlösung mit dem Affinitätschromatographiematerial in einem batch-Verfahren (Procion Red HE-3B, Sigma; gebunden an Streamline AC, Pharmacia) in Kontakt gebracht und nicht anhaftende Fraktionen im Überstand abgenommen. Die spezifische Elution der FDH erfolgte anschließend mit NAD.
Als Affinitätschromatographiematerial können dabei alle dem Fachmann für diesen Zweck in Frage kommenden Materialien genommen werden, welche in der Lage sind selektiv Dehydrogenasen zu binden. Vorzugsweise wird Sepharose benutzt, besonders bevorzugt ist der Einsatz von roter oder blauer Sepharose (A. Walsdorf, D. Forciniti, M.-R. Kula (1990) Investigation of affinity partition chromatography using formate dehydrogenase as a model. J. Chromatography 523, 103-117; U. Reichert, E. Knieps, H. Slusarczyk, M.-R. Kula, J. Thömmes (2001) Isolation of a recombinant formate dehydrogenase by pseudo affinity expanded bed adsorption, J. Biochem. Biophys. Methods, in press; N.E. Labrou, Y.D. Clonis, (1995) The interaction of *Candida boidinii* formate dehydrogenase with a new family of chimeric biomimetic dye-ligands. Arch. Biochem. Biophys. 316(1), 169-178).
Die freigesetzte FDH-Aktivität (= Ausgangsaktivität Ao) wird abschließend mit Hilfe des bekannten photometrischen Nachweisverfahrens (Lit. s.o.) bei 340 nm und 30°C in einem Mikrotiterplattenlesegerät bestimmt und mit der Proteinkonzentration bestimmt nach Bradford et al. (Beispiel 7.2) in Beziehung gesetzt.
Parallel oder anschließend kann ausgehend von den gereinigten Fraktionen auch die Stabilität bestimmt werden. Zur Überprüfung der Stabilität wurden Aliquots der Enzymproben im 96-well Format in einem PCR-Gerät (Primus 96, MWG Biotech AG) für 15 min bei einer definierten Temperatur (50°C bis 58°C) inkubiert, die sich nach der Ausgangsstabilität der Elternmutante der jeweiligen Generation richtete. Anschließend wurden die Restaktivität (A15) bei 30°C im Standardassay (Beispielen 6 und 7.2) bestimmt. Der Quotient (Ti) aus Restaktivität (A15) zu Ausgangsaktivität (Ao) ist ein Maß für die Stabilität der Enzyme und wird vorzugsweise für Stabilitätsanalysen im Screening herangezogen. Ein höherer Ti-Wert im Vergleich zu dem des Ausgangsenzyms (z.B. FDH-C23S) bedeutet, daß das untersuchte Mutein

eine höhere Stabilität aufweist.
Das Screeningverfahren zur Erhöhung der Stabilität ist in den Beispielen näher erläutert und dem Fachmann hinreichend bekannt (H. Zhao, F. Arnold (1999) Directed evolution converts subtilisin E into a functional equivalent of thermitase, Prot. Eng. 12(1), 47-53).

Mit Hilfe der oben beschriebenen Screeningsysteme können mehr als 200.000 Klone manuell in kürzester Zeit auf FDH-Aktivität und/oder Stabilität durchmustert werden.

Um zufallsmäßig Punktmutationen in das Gen der Mutante FDH-C23S bzw. FDH-C23S/C262A einzuführen, wurde die dem Fachmann bekannte Technik der fehlerhaften Polymerasekettenreaktion (Caldwell, R.C., Joyce, G.F. (1992) Randomization of Genes by PCR Mutagenesis. PCR Methods Appl. 2, 28-33) eingesetzt.
Für die erfolgreiche error prone PCR eines Genabschnitts und die Sequenzierung der Mutanten wurden fünf Primer (PBTACF1, PBTACF2) und (PBTACR1, PBTACR2, PBTACR3) konstruiert.
Zur Einstellung der Fehlerhäufigkeit wurden Manganchloridkonzentrationen von 0.15 mM und 0.5 mM im PCR-Ansatz verwendet. Mit den beiden äußeren Primern PBTACF1 und PBTACR1 bzw. PBTACR2 wurde jeweils ein 1500 bp großes Fragment der erfindungsgemäßen Gene mittels PCR-Technik amplifiziert. Beispielhaft ist die Abfolge an Basenpaaren der FDH-C23S-Mutante, die kloniert im Vektor pBTac2 als Templat in der ersten error prone PCR diente, in Seq. 1 dargestellt.

Die klonierten FDH-Gene der erfindungsgemäßen Mutanten weisen jedoch vorteilhafterweise zusätzlich am 5'-Ende eine EcoRI-Restriktionsschnittstelle und am 3'-Ende eine PstI-Schnittstelle auf, die mittels PCR unter Verwendung der Primer N-EcoRI und C-PstI an die codierende Nucleotidsequenz angefügt worden waren, um eine nachfolgende gerichtete Klonierung des Gens in den pBTac2-Vektor zu ermöglichen.

Es folgte eine Klonierung der gesamten DNA-Population von 1,1-kb großen EcoRI/PstI-Fragmenten in das zuvor mit den Restriktionsenzymen EcoRI und PstI restringierte Plasmid pBTac2. Die so entstandenen Vektoren wurden dann in E. coli transformiert, um eine Mutanten-Bibliothek zu erzeugen. Mittels des Expressionsplasmids pBTac-FDH (Fig.2) können die FDH-Muteine aus *Candida boidinii* in E. coli JM101 oder E. coli JM105 überexprimiert werden. Die Klonierungs- und Expressionstechniken sind dem Fachmann geläufig und u.a. in Sambrook et al. (s.o.) beschrieben.

Erfolgreiche Mutationen zeigten sich an folgenden Stellen:

Tabelle 2:

| Mutationen im fdh-C23S-Gen bzw. fdh-C23S/C262A- Gen, die zu einer Verbesserung der Stabilität bzw. Aktivität geführt haben. | | | |
|---|---|---|---|
| Mutation | Codon in FDH-C23S | Codon in Mutante | Mutation in Basenpaar |
| E18D | gaa | gac | 53 |
| K35R | aaa | aga | 104 |
| D149E | gat | gag | 447 |
| E151D | gag | gat | 453 |
| R178S | aga | agt | 534 |
| R178G | aga | gga | 532 |
| K206R | aaa | aga | 617 |
| F285Y | ttc | tac | 854 |
| F285S | ttc | tcc | 854 |
| T315N | act | aat | 944 |
| K356E | aaa | gaa | 1066 |

Tabelle 3:

| Mutationen in den erfindungsgemäßen Mutanten, die zu einer Verbesserung der Stabilität geführt haben (FDH-C23S (SM) und FDH-C23S/C262A (DM) waren die Template (Ausgangsmutanten) für die gerichtete Evolution). | | | | | | |
|---|---|---|---|---|---|---|
| **Bezeichnung** | **Effekt Mittlere Inaktiv. Temp.[°C]** | **Stabilitäts -erhöhung gegenüber Eltern [°C]** | **Mutation** | **Codon in FDH-C23S oder FDH-C23S/C262A** | **Codon in Mutante** | **Mutation in Basenpaar** |
| FDH-C23S/ C262A (DM) | 47 | - | C23S<br>C262A | tct<br>gct | tct<br>gct | -<br>- |
| FDH-C23S (SM) | 52 | - | C23S | tct | tct | - |
| DM-3bE10 | 51 | 4 | C23S<br>K206R<br>C262A<br>T315N<br>K356E | tct<br>aaa<br>gct<br>act<br>aaa | tct<br>aga<br>gct<br>aat<br>gaa | -<br>617<br>-<br>944<br>1066 |
| DM-2kA6 | 51 | 4 | E18D<br>C23S<br>K35R<br>R178S<br>C262A | gaa<br>tct<br>aaa<br>aga<br>gct | gac<br>tct<br>aga<br>agt<br>gct | 53<br>-<br>104<br>534<br>- |
| DM-2hG12 | 58 | 7 | E18D<br>C23S<br>K35R<br>E151D<br>R178S<br>C262A<br>F285Y | gaa<br>tct<br>aaa<br>gag<br>aga<br>gct<br>ttc | gac<br>tct<br>aga<br>gat<br>agt<br>gct<br>tac | 53<br>-<br>104<br>453<br>534<br>-<br>854 |
| SM-1kA2 | 55 | 3 | C23S<br>R178S | tct<br>aga | tct<br>agt | -<br>534 |
| SM-1eA6 | 54 | 2 | C23S<br>R178G | tct<br>aaa | tct<br>gga | -<br>532 |
| SM-2pC7 | 57 | 2 | C23S<br>D149E<br>R178S | tct<br>gat<br>aga | tct<br>gag<br>agt | -<br>447<br>534 |
| SM-4cA10 | 62 | 4 - 11 | C23<br>E151D<br>R178S<br>K206R<br>T315N | tct<br>gag<br>aga<br>aaa<br>act | tct<br>gat<br>agt<br>aga<br>aat | -<br>453<br>534<br>617<br>944 |
| SM-4fD3 | 61 | 3 - 10 | C23S | tct | tct | - |
| | | | E151D<br>R178S | gag<br>aga | gat<br>agt | 453<br>534 |
| SM-4sG4 | 59 | 1 - 8 | C23S<br>E151D<br>R178S<br>K356E | tct<br>gag<br>aga<br>aaa | tct<br>gat<br>agt<br>gaa | -<br>453<br>534<br>1066 |

Tabelle 3: (fortgesetzt)

| Mutationen in den erfindungsgemäßen Mutanten, die zu einer Verbesserung der Stabilität geführt haben (FDH-C23S (SM) und FDH-C23S/C262A (DM) waren die Template (Ausgangsmutanten) für die gerichtete Evolution). | | | | | | |
|---|---|---|---|---|---|---|
| **Bezeichnung** | **Effekt Mittlere Inaktiv. Temp.[°C]** | **Stabilitäts -erhöhung gegenüber Eltern [°C]** | **Mutation** | **Codon in FDH-C23S oder FDH-C23S/C262A** | **Codon in Mutante** | **Mutation in Basenpaar** |
| SM-4SG6 | 60 | 2 - 9 | C23S<br>E151D<br>R178S<br>K206R<br>K356E | tct<br>gag<br>aga<br>aaa<br>aaa | tct<br>gat<br>agt<br>aga<br>gaa | -<br>453<br>534<br>617<br>1066 |

Tabelle 4:

| Mutationen in den erfindungsgemäßen Mutanten, die eine Aktivitätssteigerung bewirken | | | | | | |
|---|---|---|---|---|---|---|
| **Bezeichnung** | **Effekt Katalyt. Aktivität** | **Mittlere Inaktivier. Temp. [°C]** | **Mutation** | **Codon in FDH-C23S oder FDH-C23S/ C262A** | **Codon in Mutante** | **Mutation in Basenpaar** |
| FDH-C23S/ F285S | 1,7-fach | 52 | C23s<br>F285S | tct<br>ttc | tct<br>tcc | -<br>854 |

Die Mutanten zeigten folgende verbesserte Eigenschaften:

1. Die stabilsten Muteine weisen eine um 9°C - 10°C höhere mittlere Inaktivierungstemperatur auf als die Ausgangsmutante FDH-C23S und eine um 14°C - 15°C höhere als die Ausgangsmutante FDH-C23S/C262A.

2. Die stabilsten Muteine zeigen eine bis zu 200-fach im Vergleich zur Ausgangsmutante FDH-C23S und eine bis zu 1000-fach erhöhte Halbwertszeit bei 54°C im Vergleich zur Ausgangsmutante FDH-C23S/C262A.

3. Durch die gerichtete Evolution der FDH-C23S wurde die Katalysekonstante von 3,7 $s^{-1}$ auf 6,1 $s^{-1}$, d.h. um den Faktor 1.7 erhöht. Infolgedessen erhöhte sich auch die spezifische Aktivität des Enzyms von 5,5 U/mg auf 9,1 U/mg. Daraus ergibt sich, daß die aktivere Mutante doppelt so aktiv ist wie die Ausgangsmutante FDH-C23S und das rekombinante Wildtypenzym.

4. Die Muteine können in einer Hochzelldichtefermentation des rekombinanten E. coli-Stammes JM101 somit kostengünstig bereitgestellt werden.

5. Durch Einführung der F285S-Mutation in die Mutante SM-4fD3 mittels ortsspezifischer Mutagenese wurde vorteilhafterweise eine Mutante, SM-4fD3-F285S, erzeugt, die sowohl eine gesteigerte Aktivität als auch Stabilität zeigt.

Dadurch, daß man die bereits oxidations- und aggregationsunempfindlicheren Mutanten FDH-C23S und FDH-C23S/C262A mittels gerichteter Evolution unter Verwendung von error prone-PCR und ortsspezifischer Mutagenese behandelt erhält man Informationen über bevorzugte Positionen für Aminosäureaustausche in dem Enzym und über die Art der bevorzugt zu verwendenden Aminosäure. Die derart mutierten Enzyme besitzen höhere Aktivitäten und Standzeiten und bedingen so geringere Enzymverbrauchszahlen im technischen Prozeß. Weitere Verbesserungen können durch bestimmte Kombinationen der einzelnen Mutationen erhalten werden. Mit den neuen Mutanten und deren Weiterentwicklungen ist es daher möglich, den z.B. eingangs gezeigten technischen Prozeß erheblich günstiger zu gestalten.
Für die erfindungsgemäße Anwendung können die betrachteten Enzyme in freier Form als homogen aufgereinigte Verbindungen verwendet werden. Weiterhin kann das Enzym auch als Bestandteil eines intakten Gastorganismus

eingesetzt werden oder in Verbindung mit der aufgeschlossenen und beliebig hoch aufgereinigten Zellmasse des Wirtsorganismus. Möglich ist ebenfalls die Verwendung der Enzyme in immobilisierter Form (Bhavender P. Sharma, Lorraine F. Bailey and Ralph A. Messing, "Immobilisierte Biomaterialien - Techniken und Anwendungen", Angew. Chem. 1982, 94, 836-852). Vorteilhafterweise erfolgt die Immobilisierung durch Lyophilisation (Dordick et al., J. Am. Chem. Soc. 1994, 116, 5009-5010; Okahata et al., Tetrahedron Lett. 1997, 38, 1971-1974; Adlercreutz et al., Biocatalysis 1992, 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycol-monocetylether) (Goto et al., Biotechnol. Techniques 1997, 11, 375-378). Die Verwendung als CLECs ist ebenfalls denkbar (St Clair et al., Angew Chem Int Ed Engl 2000 Jan, 39(2), 380-383).
Unter optisch angereicherten (enantiomerenangereicherten, enantiomer angereicherten) Verbindungen wird im Rahmen der Erfindung das Vorliegen einer optischen Antipode im Gemisch mit der anderen in >50 mol-% verstanden.
Eine natürliche Aminosäure ist eine solche, wie in Beyer-Walter, Lehrbuch der organischen Chemie, S. Hirzel Verlag Stuttgart, 22. Auflage, 1991, S.822f. dargestellt. Darüberhinaus sind jedoch auch entsprechende unnatürliche α-Aminosäuren angesprochen, wie z.B. in DE19903268.8 aufgeführt.
Der Organismus *Candida boidinii* ist bei der American Type Culture Collection unter der Nummer ATCC 32195 hinterlegt und öffentlich zugänglich.
Unter dem Begriff Nukleinsäuren werden sowohl DNA als auch RNA subsumiert.
Der in dieser Schrift verwendete Begriff "aktiver" wird erfindungsgemäß so verstanden, daß die spezifische Aktivität (bezogen auf die FDH-Proteinmenge) gesteigert ist.
Im Rahmen der Erfindung bezieht sich der Ausdruck in Bezug auf die Stabilität der Enzyme vorrangig auf die sogenannte Thermostabilität. Die Thermostabilität ist eine Meßgröße der physikalischen Stabilität eines Proteins. Hierunter wird im einzelnen eine Erhaltung der katalytischen Aktivität, d.h. eine Erhaltung der aktiven Konformation des Enzyms bei erhöhten Temperaturen verstanden (A.M. Klibanov, T.J. Ahern (1987) Thermal stability of proteins, In: Protein Engineering (D.L. Oxender, ed.), Alan R. Liss, Inc., 213-218). Eine erhöhte Thermostabilität wird erfindungsgemäß insbesondere so verstanden, daß die Halbwertszeit bei einer definierten Temperatur gesteigert ist (H. Zhao, F.H. Arnold (1999), Directed evolution converts subtilisin E into a functional equivalent of thermitase, Prot. Eng. 12(1), 47-53). Die Halbwertszeit ist die Zeit, nach der bei einer Inkubation bei einer definierten Temperatur noch 50% der Ausgangsaktivität vorhanden sind (Einheit = min oder h). Die Bestimmung erfolgte gemäß Beispiel 9.

Eine erhöhte Thermostabilität kann für Screeningzwecke jedoch auch aus dem sogenannten Ti-Wert hergeleitet werden. Der Ti-Wert berechnet sich aus der spezifischen Aktivität und einer Restaktivität A15 nach folgender Formel:

$$Ti = A_{15}/Ao$$

Die $A_{15}$-Aktivität ist dabei die spezifische Aktivität (bezogen auf die FDH-Proteinmenge), welche nach 15-minütigem Inkubieren der Enzyme bei einer entsprechenden Temperatur (z.B. > 50°C) noch existent ist. Eine Erhöhung des Ti-Wertes der Mutante gegenüber den Eltern deutet auf eine erhöhte Thermostabilität hin.
Unter verbesserten rec-Enzymen werden anspruchsgemäß insbesondere solche verstanden, die aktiver und/oder selektiver (im Hinblick auf die Umsetzung) und/oder unter den verwendeten Reaktionsbedingungen stabiler sind.
Von den beanspruchten Proteinsequenzen und den Nukleinsäuresequenzen werden erfindungsgemäß auch solche Sequenzen umfaßt, die eine Homologie (exclusive der natürlichen Degeneration) größer als 80 %, bevorzugt größer als 90 %, 91 %, 92 %, 93 % oder 94 %, mehr bevorzugt größer als 95 % oder 96 % und besonders bevorzugt größer als 97 %, 98 % oder 99 % zu einer dieser Sequenzen aufweisen, sofern die Wirkungsweise bzw. Zweck einer solchen Sequenz erhalten bleibt. Der Ausdruck "Homologie" (oder Identität) wie hierin verwendet, kann durch die Gleichung H (%) = [1 - V/X] x 100 definiert werden, worin H Homologie bedeutet, X die Gesamtzahl an Nukleinsäuren/Aminosäuren der Vergleichssequenz ist und V die Anzahl an unterschiedlichen Nukleinsäuren/Aminosäuren der zu betrachtenden Sequenz bezogen auf die Vergleichssequenz ist. Auf jeden Fall sind mit dem Begriff Nukleinsäuren, welche für Aminosäuresequenzen codieren, alle Sequenzen umfaßt, die nach Maßgabe der Degeneration des genetischen Codes möglich erscheinen.
Die mittlere Inaktivierungstemperatur T50 (in der Literatur oftmals auch als Tm bezeichnet) ist diejenige Temperatur, bei der nach einem definierten Inkubationszeitraum (hier 20 min) noch 50% der Ausgangsaktivität nachzuweisen ist (Einheit = °C).
Die Halbwertszeit ist die Zeit, nach der bei einer Inkubation bei einer definierten Temperatur noch 50% der Ausgangsaktivität vorhanden sind (Einheit = min oder h).
Beide Parameter stehen in einem Zusammenhang. Der eine gibt die (Thermo-)Stabilität in Abhängigkeit von der Zeit, der andere in Abhängigkeit von der Temperatur an.
Nimmt man die Inaktivierung eines Enzyms in Form einer Aktivitätsabnahme über die Zeit bei verschiedenen Temperaturen auf, so kann man aus den erhaltenen Kurven die mittlere Inaktivierungstemperatur ermitteln.
Die in dieser Schrift genannten Literaturstellen gelten als von der Offenbarung mitumfaßt.

**Ausführungsbeispiele**

**Beispiel 1: Gentechnische Methoden**

Alle hier verwendeten gentechnischen Methoden sind, wenn nicht anders vermerkt, von Sambrook et al. (1989) beschrieben und dem Fachmann bekannt. Alle Enzyme und entsprechende Puffer wurden nach Angaben der Hersteller benutzt. Die automatische Sequenzierungen mit einem ABI Sequencer (Applied Biosystems) wurden von der Firma SequiServe (Vaterstetten)durchgeführt.

**Beispiel 2: Erzeugung der Zufallsmutantenbibliothek**

Die fehlerhafte Polymerase-Kettenreaktion (error prone-PCR) wurde zur Herstellung der Mutantenbibliotheken verwendet.
Als Templat diente in der ersten Generation z.B das Plasmid pBTac-FDH-C23S, in den nachfolgenden Generationen die Plasmide mit den selektionierten stabileren bzw. aktiveren Mutanten. Die Plasmide wurden mittels einem QIAprep® Spin Miniprep Kit gemäß Herstellerangaben (Qiagen) aus E. coli isoliert. In den error prone-PCR Ansätzen wurden pBTac2-spezifische Primer als äußere Primer eingesetzt. Der PCR-Ansatz setzte sich wie folgt zusammen:

Tabelle 5:

| Zusammensetzung des error prone PCR-Ansatzes. | |
|---|---|
| Anteil | Zusammensetzung |
| 10 µl | 10x Mutagenese-Puffer (70 mM $MgCl_2$, 500 mM KCl, 0.1% (w/v) Gelatine, 100 mM Tris-HCl pH 8,3 bei 25°C) |
| 10 µl | 10x Mutagenese-dNTP-Mix (2 mM dGTP und dATP, 10 mM dTTP und dCTP) |
| 3-10µl | 10x $MnCl_2$ (5 mM $MnCl_2$) |
| 2 fmol | Templat-DNA (ca. 7,5 ng des 5,7 kb-Plasmids);<br>z.B. pBTac-FDH-C23S |
| 40 pmol | Upstream-Primer pBTacF1 |
| 40 pmol | Downstream-Primer pBTacR1 oder pBTacR2 |
| 1 µl | Taq-Polymerase (5 U $\mu l^{-1}$), Gibco |
| ad 100 µl | Millipore-Wasser |

Das verwendete PCR-Programm ist in Tabelle 6 aufgelistet.

Tabelle 6:

| error prone-PCR Programm | | |
|---|---|---|
| Schritt 1 | 95°C | 5 min |
| 2 | 94°C | 1 min |
| 3 | 50°C | 1 min |
| 4 | 72°C | X min |
| 5 | 72°C | 5 min |

Die Schritte 2 - 4 wurden 25 - 27 mal durchlaufen.
Die Annealing-Temperatur $T_A$ wurde über die DNA-Schmelztemperatur ($T_m$) der Oligonucleotide ermittelt. Die Zeit X für die Kettenreaktion der DNA-Polymerase richtete sich nach der 1 kb = 1 min-Regel. Die Ansätze wurden mit ca. 50 µl Mineralöl überschichtet.
Die Reinigung der PCR-Produkte erfolgte mit dem QIAquick® PCR Purification Kit (Qiagen) oder mit Hilfe

eines präparativen Agarosegels.

Um eine hohe Mutationsrate in der ersten Generation der Mutagenese zur Erhöhung der spezifischen Aktivität zu erzielen, wurde nach der ersten PCR 1 µl PCR-Produkt entnommen und in einer zweiten analogen PCR anstatt des FDH-C23S-Templats eingesetzt.

**Beispiel 3: Oligonucleotide**

Tabelle 7: Liste der verwendeten Oligonucleotide.

| Bezeichnung: | Verwendung: | Sequenz: |
|---|---|---|
| PBTACF1 | error prone PCR | 5´ TGC CTG GCA GTT CCC TAC TC 3´ |
| PBTACF2 | error prone PCR Sequenzierung | 5´ CGT TTC ACT TCT GAG TTC GG 3´ |
| PBTACR1 | error prone PCR | 5´ GGT ATG GCT GTG CAG GTC GT 3´ |
| PBTACR2 | error prone PCR Sequenzierung | 5´ CGA CAT CAT AAC GGT TCT GG 3´ |
| PBTACR3 | error prone PCR Sequenzierung | 5´ TCA TCG GCT CGT ATA ATG TG 3´ |
| F285S-F1 | site directed mutagenesis | 5'- GGT GAT GTT TGG TCC CCA CAA CCA GCT CCA AAG -3' |
| F285S-R1 | site directed mutagenesis | 5'- GGA GCT GGT TGT GGG GAC CAA ACA TCA CCG TA -3' |

**Beispiel 4: Ortsspezifische Mutagenese**

Zur gezielten Einführung von Punktmutationen wurde die Methode der überlappenden PCR nach Ho. et al. (1989) oder das QuickChange® Site-directed Mutagenesis Kit der Firma Stratagene laut Herstellerangaben verwendet.

**Beispiel 5: In vitro-Rekombination mittels Staggered Extension Process (StEP)**

Zur Kombination der besten Mutanten aus der Evolution der FDH-C23S und der FDH-C23S/C262A wurde

eine in vitro-Rekombination durchgeführt. Die Mutanten SM-1eA6, SM-2pC7, DM-3bE10 und DM-2hG12 wurden mittels StEP rekombiniert (H. Zhao, L. Giver, Z. Shao, J. Affholter, F. Arnold (1998) Molecular evolution by staggered extension process (StEP) in vitro recombination, Nat. Biotechnol. 16, 258-261).

Tabelle 8:

| Zusammensetzung des Rekombinationsansatzes. | |
|---|---|
| Anteil | Zusammensetzung |
| 5 µl | 10x Puffer (500 mM KCl 200 mM Tris-HCl pH 8,4) |
| 5 µl | 10x dNTP-Mix (je 2 mM dGTP, dATP, dTTP und dCTP) |
| 1,5 µl | 50 mM $MgCl_2$ |
| 0,075 pmol | Templat-DNA , jede Mutante anteilig zu gleichen Teilen |
| 7,5 pmol | Upstream-Primer PBTACF1 |
| 7,5 pmol | Downstream-Primer PBTACR1 |
| 0,5 µl | Taq-Polymerase (5 U $\mu l^{-1}$), Gibco |
| ad 50 µl | Millipore-Wasser |

Tabelle 9:

| StEP-Programm | | |
|---|---|---|
| Schritt 1 | 95°C | 10 min |
| 2 | 94°C | 30 sec |
| 3 | 50°C | 10 sec |

Die Schritte 2 - 3 wurden 80-mal durchlaufen.

Die rekombinierten FDH-DNA-Fragmente wurden zunächst mit dem Restriktionsenzym DpnI inkubiert, um die parentalen DNA-Fragmente aus den Ansätzen zu entfernen und somit den Anteil an Parentalklonen in der Mutantenbibliothek zu minimieren. Um eine Klonierung der rekombinierten Fragmente in den Vektor pBTac zu ermöglichen, wurde im Anschluß eine Restriktion mit EcoRI und PstI über Nacht bei 37°C durchgeführt. Die EcoRI- und PstI-restringierten FDH-Fragmente wurden in einem Agarosegel aufgetrennt und die FDH-Bande mittels des QIAquick® Gel Extraction Kits (Qiagen) laut Herstellerangaben isoliert und wie zuvor in pBTac2 kloniert und in E. coli JM101 transformiert. Die resultierende Mutantenbibliothek wurde mit dem in Beispiel 6 beschriebenen Screeningverfahren auf erhöhte Stabilität gescreent.

**Beispiel 6: Screeningverfahren auf erhöhte Stabilität**

Zum Screening auf erhöhte Stabilität wurde die Anzucht der Zellen und die Herstellung der zellfreien Rohextrakte gemäß Beispiel 7.2 durchgeführt. Die auf diese Weise isolierten FDH-Muteine wurden zunächst hinsichtlich ihrer Stabilität (in Anlehnung an L. Giver, A. Gershenson, P.-O. Freskgard, F. Arnold (1998) Directed evolution of a thermostable esterase, Proc. Natl. Acad. Sci. USA, 95, 12809-12813) überprüft. Dazu wurden zunächst die Ausgangsaktivitäten Ao bestimmt. 75µl zellfreier Rohextrakt wurde in einer Mikrotiterplatte mit 75 µl FDH-Assay (Zusammensetzung siehe Beispiel 7.2) versetzt und die NADH-Bildung bei 340nm und 30°C in einem Mikrotiterplattenlesegerät (Spectramax® Plus; MWG Biotech) verfolgt. Dann wurden jeweils 100 µl Rohextrakt in PCR-Probengefäße im 96er-Format überführt und 15 min in einem Thermoblock eines PCR-Gerätes (Primus 96®, Fa. MWG Biotech) bei einer definierten Temperatur inkubiert. Die Proben wurden anschließend 15 min auf Eis gekühlt und ggf. zentrifugiert (10 min, 4000rpm), um präzipitiertes Protein abzutrennen. 75 µl des Überstands wurde zur Bestimmung der Restaktivität A15 verwendet. Der Quotient aus Restaktivität A15 zur Ausgangsaktivität Ao ist ein Maß für die Stabilität der Muteine.

**Beispiel 7: Selektion der Klone mit erhöhter Aktivität**

**7.1 Qualitative Selektion**

Anzucht der Zellen

Die transformierten Zellen wurden auf $LB^{amp}$-Agarplatten ausgestrichen und über Nacht bei 37°C inkubiert,

so daß die Kolonien einen Durchmesser von ca. 1 mm hatten.

Einbetten der Zellen

Anschließend wurden die Kolonien mit einer Lage Agar (1,6% Agar, 100 mM $KP_i$ pH 7,5, 0,2% Triton X-100, 10 mM EDTA) überschichtet. Die Agar-Lösung mußte vor dem Überschichten auf eine Temperatur von höchstens 65°C abgekühlt sein. Nach dem Erstarren des Agars wurde fünfmal mit Aufschlußlösung (100 mM $KP_i$ pH 7,5, 0,2% Triton X-100, 10 mM EDTA) und fünfmal mit Waschlösung (100 mM $KP_i$ pH 7,5) gewaschen (5 mm Flüssigkeitshöhe in den Platten).

Aktivitätsfärbung

Zur Färbung wurden die Platten mit Färbelösung-1 (1,25 M Formiat; 0,2 g $l^{-1}$ Phenazinethosulfat; 2 g $l^{-1}$ Nitrotetrazoliumblauchlorid; 100 mM $KP_i$ pH 7,5) 2 mm hoch überschichtet und 10 min im Dunkeln geschüttelt. Anschließend wurde die Färbelösung-2 (50 mM NAD) in einem Verhältnis von 1 ml Färbelösung-2 pro 100 ml Färbelösung-1 zugegeben und im Dunkeln ca. 15 min geschüttelt, bis die Höfe deutlich zu erkennen waren.

Anschließend wurde die Lösung abgeschüttet, die Platten zweimal kurz mit Wasser gewaschen und zum Trocknen offen aufgestellt. Nachdem die Platten trocken waren, konnten die Klone mit sterilen Zahnstochern in 96-well Platten, die mit 200 µl $LB^{amp}$-Medium gefüllt waren, überführt werden. Die Anzucht der Zellen erfolgte über Nacht bei 37°C auf einem Schüttler. Diese Platten dienten als Master-Platten für die folgende quantitative Selektion.

**7.2 Quantitative Selektion**

Anzucht der Zellen

Zur Anzucht wurden sterile deep-well-Platten im 96er-Format verwendet. Sie wurden mit 1,2 ml $LB^{amp}$-Medium gefüllt und mit 50 µl Zellsuspension der Master-Platten angeimpft. Nach einer Wachstumsphase von 4 h bei 37°C wurden die Zellen mit 50 µl einer IPTG-Lösung (20 mM IPTG in A.dest, sterilfiltriert) induziert und bei 30°C über Nacht bei 140 rpm geschüttelt.

Zellaufschluß, Herstellung der zellfreien Rohextrakte

Die 96er deep-well-Platten wurden anschließend zentrifugiert (1.600 g, 15 min, 20°C), der Überstand abgegossen, die Zellen in 500 µl Puffer-Lösung (10 mM $KP_i$ pH 7,5) resuspendiert und 1/10 Vol. Aufschlußlösung (2% Triton X-100, 10 mM $KP_i$ pH 7,5, 100 mM EDTA) zugegeben und 1 h bei 37°C geschüttelt. Danach wurden die Platten zentrifugiert (1.600 g, 15 min, 20°C). 200 µl des Überstandes wurden danach abgenommen und auf das Affinitätschromatographiematerial gegeben.

Reinigung mittels Affinitätschromatographie

Die rote Sepharose (Procion Red HE-3B, DyStar, Frankfurt; gebunden an Streamline AC, Pharmacia) (U. Reichert, E. Knieps, H. Slusarczyk, M.-R. Kula, J. Thömmes (2001) Isolation of a recombinant formate dehydrogenase by pseudo affinity expanded bed adsorption, J. Biochem. Biophys.
Methods, in press) wurde vor Gebrauch regeneriert. Dazu wurde sie mit Regenerationslösung-1 (1 M NaCl, 25% Ethanol) und anschließend mit Regenerationslösung-2 (4 M Harnstoff, 0,5 M NaOH) gewaschen. Danach wurde sie mit 10 mM $KP_i$ pH 7,5 äquilibriert.
200 µl des Überstands der Zellaufschlußlösung wurden in eine 96-well PCR-Platte (Roth) gegeben, in der sich 10 µl regenerierte Rote Sepharose befanden. Die PCR-Platte wurde dann mit PCR-Strips verschlossen und für 1 h bei 20°C langsam auf einem Überkopfschüttler geschüttelt. Danach wurde die Platte kurz zentrifugiert (1.600 g, 1 min, 20°C) und der Überstand abgesaugt. Die Rote Sepharose wurde anschließend zehnmal mit der Waschlösung-1 (40 mM NaCl, 40 mM $NaHSO_4$, 100 mM $KP_i$ pH 7,5) und zweimal mit der Waschlösung-2 (100 mM $KP_i$ pH 7,5) gewaschen. Die Elution erfolgte mit der Elutionslösung (15 mM NAD, 100 mM $KP_i$ pH 7,5). Dazu wurde 200 µl der Elutionslösung zugegeben, die PCR-Platte verschlossen und für 1 h bei 20°C langsam auf einem Überkopfschüttler geschüttelt. Danach wurde die Platte kurz erneut zentrifugiert (1.600 g, 1 min, 20°C) und der Überstand für die Bestimmung der Volumenaktivität und der Proteinkonzentration verwendet.

Bestimmung der spezifischen Aktivität

Die Volumenaktivität wurde in Mikrotiterplatten mit Hilfe des Mikrotiterplatten-Photometers Thermomax plus (Molecular Devices) bestimmt. Dazu wurde in 75 µl Puffer (100 mM $KP_{i,}$ pH 7,5) 25 µl der eluierten Formiatdehydrogenase gegeben. Kurz vor der Messung werden 100 µl Aktivitäts-Assay (0,5 M Na-Formiat, 4 mM NAD, 100 mM $KP_i$, pH 7,5) pro Well zugegeben und die Volumenaktivität bestimmt.

Die Proteinkonzentrationsbestimmung erfolgte ebenso in Mikrotiterplatten. Zur Eichung wurde eine Eichkurve mit BSA (Fraktion V) herangezogen. Zu 50 µl der eluierten Formiatdehydrogenase wurden 150 µl 1,2x Bradfordlösung zugegeben und die Proteinkonzentration bestimmt.
Die Berechnung der spezifischen Aktivität $V_{max}$ [U $mg^{-1}$] erfolgt durch Division der Volumenaktivität mit der Proteinkonzentration. Mit Hilfe des bekannten Molekulargewichtes der Formiatdehydrogenase von 40.370 g $mol^{-1}$ kann die Wechselzahl $k_{cat}$ [$s^{-1}$] berechnet werden:

$$k_{cat} = 6x10^{-4} \text{ Vmax x Mw}$$

**Beispiel 8: Aufreinigung der FDH-Muteine aus E. coli JM101**

Die Aufreinigung der heterolog überexprimierten FDH-Muteine erfolgte nach Zellaufschluß in einem Schritt:

1. Zellaufschluß: 40%ige Zellsuspension mit 50 mM Kpi (pH 7,5) wurde mittels Ultraschall aufgeschlossen.

2. Affinitätschromatographie im Batch-Verfahren mit Roter Sepharose analog zum zuvor beschriebenen Verfahren.

**Beispiel 9: Bestimmung der Halbwertszeit**

Zur Bestimmung der Halbwertszeit wurden die Enzymlösungen im 100 mM Kpi, pH 7.5, auf gleiche Volumenaktivitäten und Volumina gebracht, um gleiche Oberflächen-Volumen-Verhältnisse zu gewährleisten. Die Volumenaktivität gibt die photometrisch bestimmte Enzymaktivität pro Volumen Enzymlösung (Units / ml) an. Ein Unit ist definiert als die Menge Enzym, die die Reduktion von 1 µmol NAD, gemessen an der Extinktionsänderung bei 340 nm, pro Minute bei 30°C und pH 7.5 ermöglicht. Die Proben wurden z.B. bei einer definierten Temperatur im Bereich zwischen 46°C und 62°C inkubiert und zu verschiedenen Zeitpunkten Aliquots zur Bestimmung der Volumenaktivität entnommen. Bei logarithmischer Auftragung der Volumenaktivität kann durch lineare Regression die Steigung *k* bestimmt werden, mit der die Halbwertszeit $t_{1/2}$ berechnet werden kann:

$$t_{1/2} = 1n2 / k .$$

**Patentansprüche**

1. Gegenüber der Wildtyp-rec-FDH und dem nativen Wildtypenzym aus *Candida boidinii* stabilere und/oder aktivere Mutanten,
**dadurch gekennzeichnet, daß**
diese Mutanten den Aminosäureaustausch C23S oder C23S/C262A und weiterhin einen oder mehrere der folgenden Aminosäureaustausche aufweisen:E18D, K35R, D149E, E151D, R178S, R178G, K206R, F285Y, F285S, T315N, K356E.

2. Aminosäuresequenzen mit FDH-Aktivität, welche gegenüber der Wildtyp-rec-FDH und dem nativen Wildtypenzym aus *Candida boidinii* stabiler und/oder aktiver sind, aufweisend einen oder'mehrere der folgenden Aminosäureaustausche:
18D, 35R, 149E, 151D, 178S, 178G, 206R, 285Y, 285S, 315N, 356E, wobei die Austausche an den entsprechenden äquivalenten Positionen in der Sequenz erfolgen.

3. Nukleinsäuren aufweisend eine Sequenz codierend für eine rec-FDH gemäß Anspruch 1 und/oder 2.

4. Plasmide, Vektoren, Mikroorganismen aufweisend eine oder mehrere Nukleinsäuren nach Anspruch 3.

5. Primer zur Herstellung der Nukleinsäuren nach Anspruch 3 mittels PCR.

6. Verfahren zur Herstellung von verbesserten rec-FDHs ausgehend von Nukleinsäuren codierend für eine rec-FDH nach Anspruch 1,
**dadurch gekennzeichnet, daß** man

   a) die Nukleinsäuren einer Mutagenese unterwirft,
   b) die aus a) erhältlichen Nukleinsäuren in einen geeigneten Vektor kloniert und diesen in ein geeignetes Expressionsystem transferiert und
   c) die gebildeten verbesserten Proteine detektiert und isoliert.

7. rec-FDHs oder diese codierende Nukleinsäuren erhältlich nach Anspruch 6.

8. Verwendung der rec-FDHs gemäß Anspruch 1 oder 6 in einem Verfahren zur Herstellung von chiralen Verbindungen, insbesondere Alkoholeen und Aminosäuren.

9. Verwendung der Nukleinsäuren gemäß Anspruch 3 oder 7 zur Herstellung von Ganzzellkatalysatoren.

10. Ganzzellkatalysatoren aufweisend ein kloniertes Gen für eine Dehydrogenase und ein kloniertes Gen für eine rec-FDH.

11. Ganzzellkatalysator gemäß Anspruch 10,
**dadurch gekennzeichnet, daß**
es sich um eine rec-FDH aus Candida boidinii handelt.

12. Ganzzellkatalysator gemäß Anspruch 10 und/oder 11,
**dadurch gekennzeichnet, daß**
es sich um eine rec-FDH gemäß Anspruch 1 und/oder 2 handelt.

**13.** Verfahren zur Identifizierung aktiverer Mutanten einer NAD- oder NADP-abhängigen Dehydrogenase aufweisend ein quantitatives Screeningverfahren zur Bestimmung der Aktivität, welches im einzelnen folgende Schritte aufweist:

a) Zellaufschlußlösung der zu vergleichenden Mutanten wird in gleichen Aliquots mit jeweils gleichen Mengen Affinitätschromatographiematerial zusammengebracht,
b) Trennen des Affinitätschromatographiematerials von den nicht haftenden Bestandteilen,
c) Eluieren der an dem Affinitätschromatographiematerial haftenden Muteine,
d) Bestimmung der Volumenaktivität und Proteinkonzentration und daraus der spezifischen Aktivität.

**14.** Verfahren nach Anspruch 13,
**dadurch gekennzeichnet, daß**
es sich bei den Enzymen um eine FDH handelt.

Fig. 1:

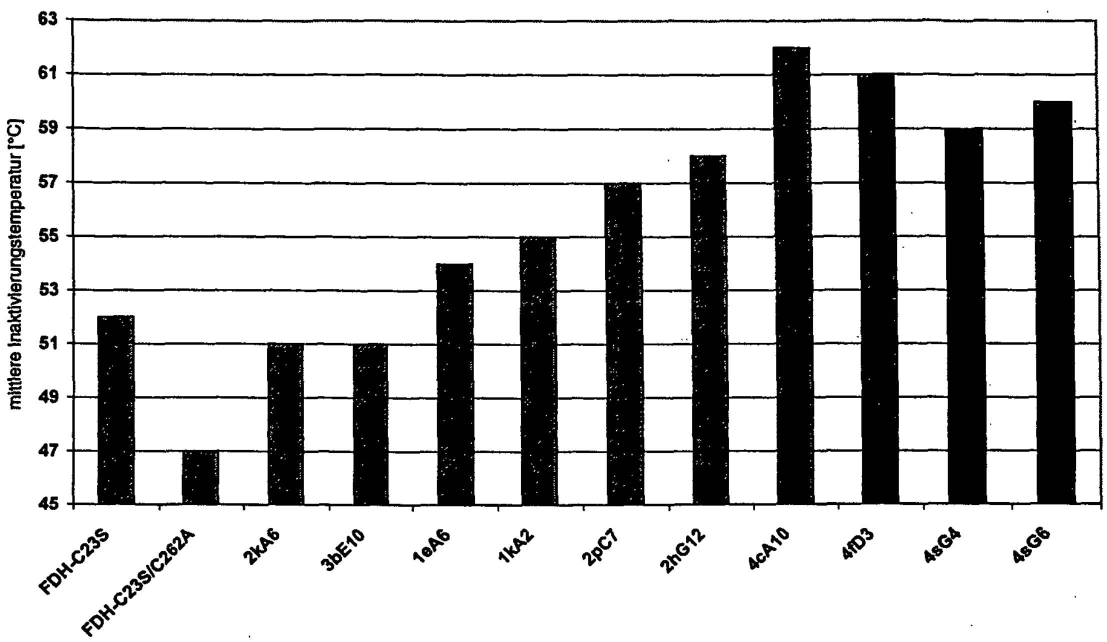
mittlere Inaktivierungstemperatur [°C]
63
61
59
57
55
53
51
49
47
45
FDH-C23S
FDH-C23S/C262A
2kA6
3bE10
1eA6
1kA2
2pC7
2hG12
4cA10
4fD3
4sG4
4sG6

Fig. 2

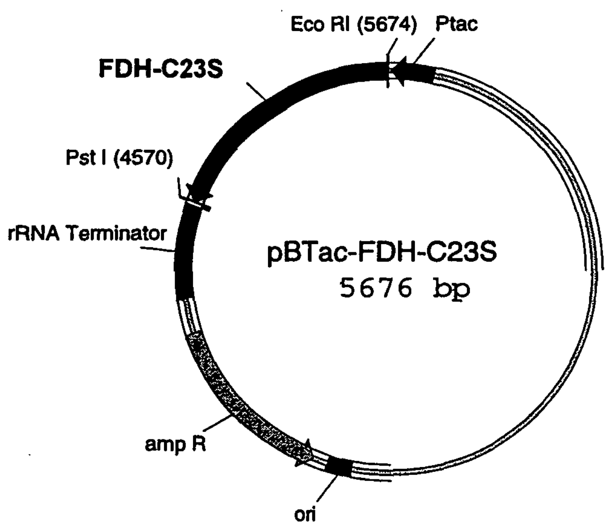
Eco RI (5674)
Ptac
FDH-C23S
Pst I (4570)
rRNA Terminator
pBTac-FDH-C23S
5676 bp
amp R
ori

Fig. 3:

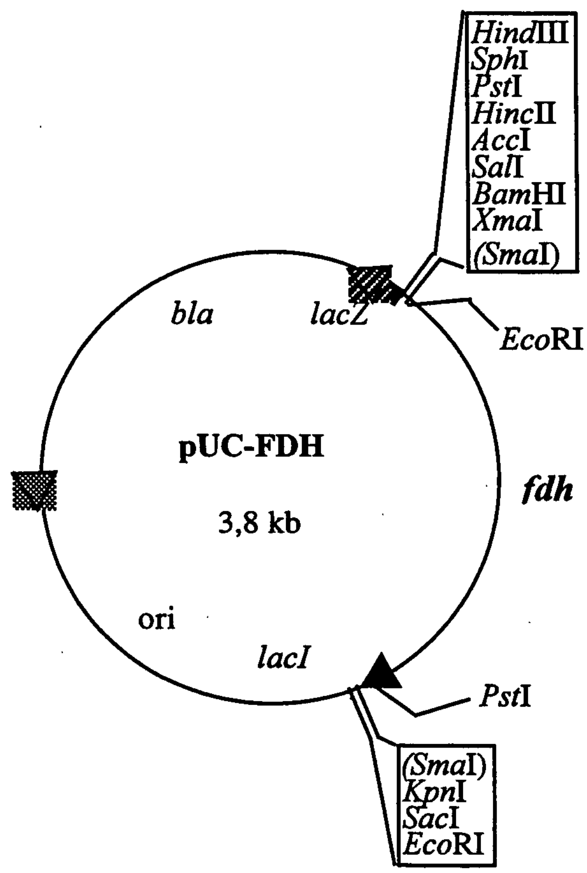
HindIII
SphI
PstI
HincII
AccI
SalI
BamHI
XmaI
(SmaI)
bla
lacZ
EcoRI
pUC-FDH
3,8 kb
fdh
ori
lacI
PstI
(SmaI)
KpnI
SacI
EcoRI